(19) Europäisches Patentamt
European Patent Office
Office européen des brevets

(11) Numéro de publication: **0 378 468 B1**

# (12) FASCICULE DE BREVET EUROPEEN

(45) Date de publication de fascicule du brevet: **12.10.94**

(21) Numéro de dépôt: **90400056.9**

(22) Date de dépôt: **09.01.90**

Le dossier contient des informations techniques présentées postérieurement au dépôt de la demande et ne figurant pas dans le présent fascicule.

(51) Int. Cl.5: **C07D 487/04**, C07D 471/04, C07D 401/06, C07D 403/06, C07D 401/14, C07D 207/04, C07D 211/08, C07D 223/04, A61K 31/505, A61K 31/495, //(C07D471/04,239:00,221:00), (C07D487/04,239:00,235:00)

## (54) Dérivés des bisarylalcènes, leurs procédés de préparation et les compositions pharmaceutiques qui les contiennent.

(30) Priorité: **10.01.89 FR 8900213**

(43) Date de publication de la demande: **18.07.90 Bulletin 90/29**

(45) Mention de la délivrance du brevet: **12.10.94 Bulletin 94/41**

(84) Etats contractants désignés: **AT BE CH DE DK ES FR GB GR IT LI LU NL SE**

(56) Documents cités:
**EP-A- 0 110 435**
**EP-A- 0 266 246**

(73) Titulaire: **ADIR ET COMPAGNIE**
**22, rue Garnier**
**F-92201 Neuilly sur Seine (FR)**

(72) Inventeur: **Lavielle, Gilbert**
**1 Avenue Lilly**
**F-78170 La Celle Saint Cloud (FR)**
Inventeur: **Colpaert, Francis**
**36bis Boulevard Carnot**
**F-78110 Le Vesinet (FR)**
Inventeur: **Laubie, Michel**
**35 Avenue Foch**
**F-92420 Vaucresson (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**CHEMICAL ABSTRACTS**, vol. 105, no. 3, 1986, page 49, résumé no. 18138x,Columbus, Ohio, US; C. KORSTANJE et al.: "Characterization of flufylline,fluprofylline, ritanserin, butanserin and R 56413 with respect to in-vivo alpha1-,alpha2- and 5-HT2-receptor antagonism and in vitro affinity for alpha1-, alpha2- and 5-HT2-receptors: comparison with katanserin",& J. PHARM. PHARMACOL. 1986, 38(5), 374-9

## Description

La présente invention a pour objet des nouveaux dérivés des bisarylalcènes, leurs procédés de préparation et les compositions pharmaceutiques qui les contiennent.

Certains dérivés de la morpholinyl, pyrrolidinyl, pipéridyl, pipérazinyl, perhydrothiazinyl ou cyclohexenyl alkyl-1 ou oxoalkyl-1 diphénylméthylène-4 pipéridine, ayant des propriétés anticholinergiques sont déjà décrits (Brevets US 4.540.780; US 4.584.301; US 4.640.925; US 4.666.905). On connait aussi des dérivés de diphénylméthyl-4 ou de diphénylméthylène-4 pipéridine substitués en 1 par des oléfines, des alcools, des cétones ou des oximes (Brevets US 4.180.583; US 3.878.217; DE 2.303.305; DE 2.303.245; DE 2.303.246; US 3.922.276). Ces derniers composés sont doués de propriétés antihistaminiques, antiallergiques et bronchodilatatrices, ou sont des anti-inflammatoires et des tranquillisants. Des dérivés de [-(diphénylméthylène-4 pipéridyl-1) alkyl]-1 benzimidazolone-2 (Demande de Brevet EP 181.793) de [-(diphénylméthylène-4 pipéridinyl-1) alkyl]-3 imidazo [4,5-b] pyridinone-2 (Demande de Brevet EP 266.246), ou de (bisarylméthylène-4 pipéridinyl-1) alkyl pyrimidinones (Demande de Brevet EP 110.435) sont connus comme étant des antagonistes de la sérotonine, comme il a été montré par C. KORSTANJE et coll. (J. Pharm. Pharmacol., 38(5), 374-379, 1986). Des dérivés arylalkyle ou arylalcène des pyrrolidines, pipéridines ou d'homo-pipéridines substitués au niveau de l'azote avec des chaînes latérales contenant des hétéroatomes sont décrits dans les demandes de brevet EP 228.893 et EP 235.463 comme ayant des activités cardiovasculaires, antihistaminiques et antisécrétoires. Des dérivés de la (diphényl-1,1 alcényl-1)-1 pipérazine ayant des propriétés anti-dépres-sives sont aussi connus (Demande de Brevet FR 87.05311).

Les composés de la présente invention, qui sont des dérivés des bisarylalcènes de structure originale, possèdent des propriétés pharmacologiques remarquables. En effet, ils sont des antagonistes de la sérotonine au niveau $5HT_2$ sans composante adrénolytique $\alpha_1$. De plus, les composés de l'invention sont capables d'antagoniser spécifiquement un symptôme complexe induit chez l'animal par l'injection du 5-hydroxytryptophane, ce qui laisse présager que ces nouveaux composés sont également antagonistes de la sérotonine au niveau des récepteurs du type $5HT_1$. Les composés de l'invention se démarquent nettement des autres dérivés de bisarylalcènes déjà décrits dans la littérature.

La présente invention a plus particulièrement pour objet les dérivés des bisarylalcènes de formule I :

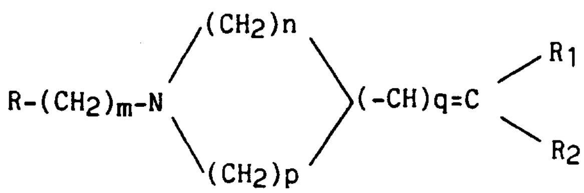

(I)

dans laquelle

- m représente un nombre entier de 2 à 4,
- n et p identiques ou différents représentent un nombre entier égal à 1, 2 ou 3, à la condition que la somme de n et de p soit supérieure ou égale à 3, et inférieure ou égale à 5,
- q représente 0 ou 1
- R représente un radical tétrahydro-1,2,3,4 quinazolinyle-3 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-1,3 hexahydro-1,3,4,6,11,11a-2H pyrazino [1,2-b] isoquinolyle-2, un radical dihydro-1,2 oxo-1 phtalazinyle-2 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-2,6 pipérazinyle-1 de formule W :

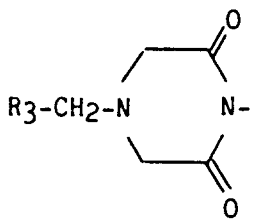

(W)

(dans laquelle $R_3$ représente un radical pyridyle-2 ou un radical phényle éventuellement substitué avec un ou plusieurs atomes d'halogène ou des radicaux alkyle ou alcoxy de 1 à 6 atomes de carbone),

un radical de formule Z :

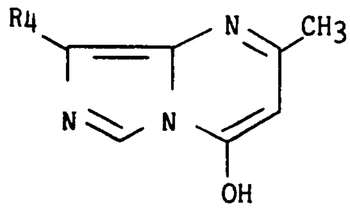

(Z)

(dans laquelle $R_4$ représente un radical carbamoyle, un radical cyano, un radical hydroxycarbonyle, ou un radical alcoxycarbonyle de 1 à 6 atomes de carbone),

ou un radical de formule Y :

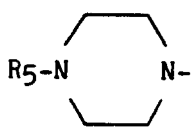

(Y)

(dans laquelle $R_5$ représente un radical pyrimidinyl-2, un radical isoquinolyl-1, un radical quinolyl-2, un radical pyridyl-2, un radical benzyle -éventuellement substitué par un radical alkyle de 1 à 6 atomes de carbone comportant un ou plusieurs atomes d'halogène-, un radical thiazolyl-2 -éventuellement substitué par un ou plusieurs radicaux alkyle de 1 à 6 atomes de carbone ou par un radical phényle-, ou un radical benzothiazolyl-2),

- $R_1$ et $R_2$

soit

identiques ou différents représentent chacun un radical phényle substitué par un ou plusieurs atomes d'halogène ou par un ou plusieurs radicaux alkyle ou alcoxy contenant de 1 à 6 atomes de carbone, soit

$R_1$ représente un radical phényle et $R_2$ un radical pyridyle-2 (chacun de ces deux radicaux pouvant être substitué par un ou plusieurs atomes d'halogène ou par un ou plusieurs radicaux alkyle ou alcoxy contenant de 1 à 6 atomes de carbone),

soit

$R_1$ et $R_2$ forment ensemble avec l'atome de carbone auquel ils sont attachés un radical fluorène,

leurs stéréoisomères possibles et leurs sels d'addition à un acide minéral ou organique pharmaceutiquement acceptable.

La présente invention a également pour objet un procédé de préparation de composés de formule générale I, caractérisé en ce que :

<u>soit</u>

l'on condense

ou bien

une amine de formule générale II

RH (II)

dans laquelle R a la même signification que pour la formule I avec un composé de formule générale III :

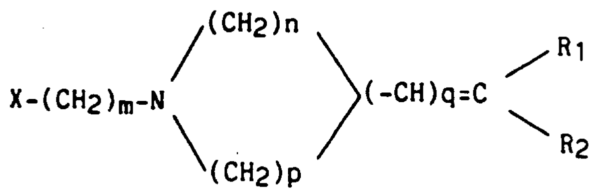

(III)

dans laquelle X représente un atome d'halogène, un radical mésyloxy, ou un radical tosyloxy et m, n, p, q, et $R_1$ et $R_2$, ont la même signification que précédemment, pour former les composés de formule I,

ou bien

une amine de formule IV :

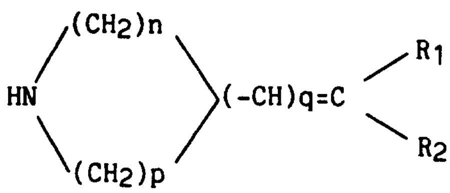

(IV)

dans laquelle $R_1$, $R_2$, n, p, q ont la même signification que pour la formule I,

- avec un composé de formule V :

$R - (CH_2)_m - X$ (V)

dans laquelle X, R et m ont la même signfication que précédemment pour former les composés de formule I,

**soit**

l'on cyclise un dérivé de l'amino-4 imidazole avec un composé de formule VI:

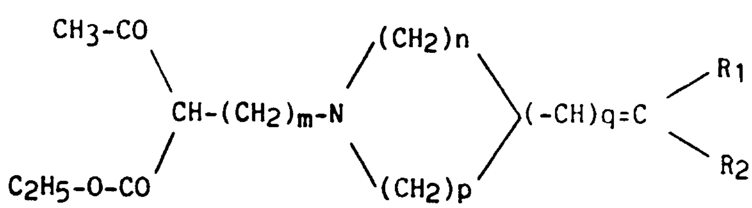

(VI)

dans laquelle m, n, p, q, $R_1$, $R_2$ ont la même signfication donnée pour la formule I,

pour former les composés de la formule I dans laquelle R comprend un groupement imidazo [1,5a] pyrimidine et m, n, p, q, $R_1$, $R_2$ ont la signification indiquée pour la formule I,

lesquels ensuite

si l'on désire, sont séparés en leurs stéréoisomères possibles ou/et salifiés par un acide organique ou minéral, pharmaceutiquement acceptable, pour former les sels d'addition correspondants.

Certains composés répondant à la formule générale II comme par exemple la (pyrimidinyl-2)-1 pipérazine, la (pyridyl-2)-1 pipérazine et l'amino-4 carbamoyl-5 imidazole, sont des produits commerciaux (Aldrich R). L'hexahydro-1,3,4,6,11,11a-2H-pyrazino [1,2-b] isoquinoléine dione-1,3, composé répondant aussi à la formule II peut être préparé par chauffage de l'acide carboxy-3 tétrahydro-1,2,3,4 isoquinolyl-2 acétique dans le formamide. Ce dernier composé est préparé par l'action de l'acide chloro-2 acétique sur l'acide tétrahydro-1,2,3,4 isoquinoléine carboxylique-3. La (quinolyl-2)-1 pipérazine est obtenue comme décrit dans le brevet US 3,743,732. L'(isoquinolyl-1)-1 pipérazine est obtenue comme décrit dans le brevet US 3,932,412.

Les composés de formule V sont obtenus en traitant les composés de formule générale II soit avec un bromo chloro alcane de formule VII :

$Br(CH_2)_mCl$ (VII)

dans laquelle m a la même signification que pour la formule I, soit avec des halohydrines de formule VIII :

$Hal(CH_2)_mOH$ (VIII)

dans laquelle m a la même signification que pour la formule I et Hal représente un atome d'halogène. Les alcools ainsi obtenus sont ensuite transformés en dérivés de formule V par des méthodes classiques.

Les amines de formule IV correspondent aux amines $IV_a$-$IV_d$.

Les amines de formule IVa :

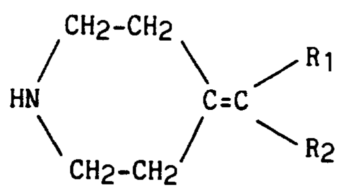

$(IV_a)$

dans laquelle $R_1$ et $R_2$ ont la même signification que pour la formule I, sont préparées à partir de dérivés halogènes en 4 d'alkyl-1 pipéridine et des cétones de formule IX :

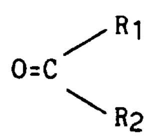

(IX)

dans laquelle $R_1$ et $R_2$ ont la même signification que pour la formule I, en présence de magnésium selon des procédés connus (Réaction de Grignard). Les alcools tertiaires issus de cette réaction sont ensuite soumis à une déshydratation. Les bis(aryl)méthylène-4 alkyl-1 pipéridines, ainsi obtenues, sont ensuite désalkylées par des procédés classiques.

Les amines de formule $IV_b$ :

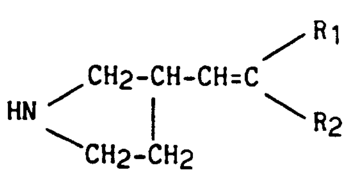

$(IV_b)$

dans laquelle $R_1$ et $R_2$ ont la signification indiquée pour la formule I,

sont préparés à partir de (benzyl-1 pyrrolidinyl-3) acétonitrile qui est transformé en (benzyl-1 pyrrolidinyl-3)-2 acétate d'éthyle. Ce composé est ensuite soumis à l'action des halogénures d'aryle magnésium et les alcools tertiaires, issus de la réaction, donnent après déshydratation et désalkylation les amines secondaires attendues.

Les composés de formule $IV_c$ :

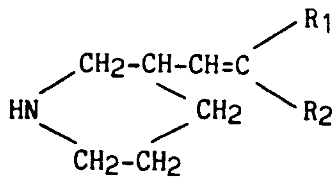

(IVc)

dans laquelle $R_1$ et $R_2$ ont la signification indiquée pour la formule I,

sont préparés à partir de la chlorométhyl-3 méthyl-1 pipéridine. Ce composé par des méthodes classiques est transformé en chlorure de (méthyl-1 pipéridyl-3) méthyl magnésium. Ce dernier est ensuite condensé avec une cétone de formule IX pour former des dérivés de (méthyl-1 pipéridyl-3)-2 bisaryl-1,1 éthanol, qui, après déshydratation et déméthylation donnent les composés de formule $IV_c$.

Les amines de formule $IV_d$ :

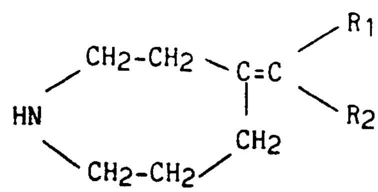

(IVd)

dans laquelle $R_1$ et $R_2$ ont la signification indiquée pour la formule I,

sont préparées à partir du N,N-diméthyl amino-4 butyronitrile. Ce composé est condensé avec le chloro-3 iodo-1 propane pour former le (N,N-diméthyl amino-2 éthyl)-2 chloro-5 pentanenitrile qui après cyclisation donne la cyano-4 méthyl-1 perhydroazépine. Les amines de formule $IV_d$ sont synthétisées à partir de ce nitrile selon le procédé décrit pour les dérivés $IV_b$.

Les composés de formule III sont préparés en traitant les amines de formule IV soit avec des bromochloroalcanes de formule VII soit avec des halohydrines de formule VIII, qui donnent dans une première étape des alcools transformables en dérivés de formule III par des méthodes classiques.

Les composés de formule VI sont préparés par condensation des composés de formule III sur l'acétoacétate d'éthyle (Ann.Rep.Sankyo Res.Lab.,(1977),29,p.75-98).

La condensation des amines de formule II ou IV avec les composés de formule III ou V est réalisée dans un solvant organique polaire en présence de sels minéraux tels que le carbonate de sodium et l'iodure de sodium à une température comprise entre 40 °C et 100 °C.

La cyclisation de l'amino-4 carbamoyl-5 imidazole avec les composés de formule VI s'effectue à chaud et en présence d'acide phosphorique.

Parmi les acides pharmaceutiquement acceptables pour la préparation des sels d'addition aux composés de formule générale I, on peut citer les acides chlorhydrique, phosphorique, fumarique, citrique, oxalique, sulfurique, ascorbique, tartrique, maléïque, mandélique, méthanesulfonique etc...

Les composés de la présente invention possèdent des propriétés pharmacologiques fort intéressantes. En effet, ils ont été soumis à divers essais pharmacologiques qui ont montré leurs activités antagonistes de l'histamine et de la sérotonine au niveau des récepteurs $5HT_2$, sans composante adrénolytique $\alpha_1$. Les composés de l'invention inhibent aussi d'une manière puissante les symptomes complexes et caractéristiques induits chez l'animal par l'injection de 5-hydroxytryptophane. Les composés de la présente invention sont donc également antagonistes de la sérotonine au niveau des récepteurs de type $5HT_1$ (J.Ph.Ex.Ther.,-(1984), 228, N°1,p.133-139). Les essais in vivo ont aussi démontré que les composés de l'invention sont très bien absorbés par voie orale, ce qui constitue un avantage considérable lors de leurs applications en thérapeutique.

Les propriétés antihistaminiques des composés de l'invention permettent leurs utilisations comme agents antiallergiques, antiprurigineux pour le traitement des voies respiratoires telles que rhinites, rhumes de foins, pour le traitement de l'asthme, et d'oedème de Quincke.

Les dérivés de l'invention plus spécifiquement actifs comme antagonistes des récepteurs à la sérotonine au niveau central et tout particulièrement des récepteurs $5HT_2$ et $5HT_1$, peuvent être utilisés pour lutter contre certains effets indésirables de ces médiateurs. Ils trouvent leurs applications plus spécialement dans l'anxiété et la dysthymie (Ceulemans DLS, Hoppenbrouwers M.L., Gelders Y.G., Reyntjens A.J.M., Pharmacopsychiat., (1985),18,p.303-305 et Le Bars, Neuronal Serotonin Eds Osborne. NN and Hamon M., John Wiley and Sons Ltd, N.Y., (1988),p.171-229), dans la dépression et le stress (Anisman H and Zacharko R.M., Behav.Brain. Scienc.,(1982),5,p.89-137 et Blier P., de Montigny C. and Chaput Y., J.Clin.Psychopharmacol.,(1987),7,p.245-335), la douleur (Jacobs B.L. and Trulson M.E., TINS, (1979)-Novem., p.276-280), les troubles de mémoire (Markianos M., Hadjikonstantinou and Bistolaki E., Acta Neurol. Scand., (1982),66,p.267-275), la maladie de Parkinson (Le Bars, Neuronal Serotonin Eds. Osborne NN and Hamon M, John Wiley and Sons Ltd N.Y.,(1988),p.171-229), la schizophrénie (Borison R.L., Havdala H.S. and Diamond B.I., Comms. Psychopharmacol.,(1978),2,p.209-214 et Iversen S.D., Neuropharmacol.,-(1984),23,p.1553-1560) et la migraine (Fozard J.R. et Gray J.A., TIPS,(1989),10,p. 307-309).

L'invention s'étend aussi aux compositions pharmaceutiques renfermant comme principe actif au moins un composé de formule générale I, ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, suppositoires, solutions injectables ou buvables.

La posologie peut varier largement en fonction de l'âge, du poids du patient, de la nature et de la sévérité de l'affection ainsi que de la voie d'administration. D'une manière générale, la posologie unitaire s'échelonnera entre 0,5 et 100 mg et la posologie journalière, utilisable en thérapeutique humaine, entre 0,5 mg et 300 mg.

La voie d'administration préférée est la voie orale ou parentérale.

Les exemples suivants, donnés à titre non-limitatif, illustrent l'invention.

Les points de fusion ont été mesurés selon la technique Micro-Köfler.

Les spectres de résonance magnétique nucléaire du proton (RMN-$^1H$) ou de carbone $^{13}C$ (RMN-$^{13}C$) des composés de formule générale I ont été enregistrés selon le cas à 60, 200 et 400 MHz et sont indiqués dans le Tableau I.

**EXEMPLE 1**

**Chlorhydrate de la {[[bis (fluoro-4 phényl) méthylène]-4 pipéridino]-2 éthyl}-3 carbamoyl-8 hydroxy-4 méthyl-2 imidazo [1,5-a] pyrimidine**

STADE A

Chlorhydrate de la [bis (fluoro-4 phényl) méthylène]-4 chloroéthyl-1 pipéridine

Dissoudre 0,31 mole d'oxyde d'éthylène dans une solution de 0,28 mole de [bis(fluoro-4 phényl)-méthylène]-4 pipéridine (préparée selon le procédé décrit dans le brevet US 3,922,276) dans un litre de méthanol anhydre à -10 °C compléter la réaction par agitation pendant 15 heures à -20 °C puis pendant 3 heures à 45 °C. Concentrer sous vide. Purifier par chromatographie sur 140 g de silice 230-400 mesh en utilisant comme éluant un mélange de dichlorométhane et de méthanol (95:5 V/V). L'huile obtenue est solubilisée dans un litre de toluène anhydre, et ensuite ajouter 0,27 mole de chlorure de thionyle. Porter à reflux pendant 30 min pour compléter la réaction. Refroidir à 20 °C et filtrer le précipité formé.

Rendement : 90%

Spectre de résonance magnétique nucléaire du proton (200 MHz, solvant $CDCl_3$) : 2,5-3,3 ppm,m,6H; 3,4 ppm,t,2H; 3,5-3,9 ppm,m,2H; 4,1 ppm,t,2H; 6,8-7,4 ppm,m,8H.

STADE B

Acétyl-2 {[bis(fluoro-4 phényl)méthylène]-4 pipéridino}-4 butyrate d'éthyle

A 0°C, ajouter 0,182 mole d'acétoacétate d'éthyle à une suspension contenant 0,182 mole d'hydrure de sodium dans 800 ml de tétrahydrofuranne. Maintenir le milieu réactionnel une heure à 20°C puis ajouter 0,182 mole d'iodure de sodium. Refroidir à 0°C et additionner 0,182 mole de [bis (fluoro-4 phényl)-méthylène]-4 chloroéthyl-1 pipéridine. Porter à reflux pendant 12 heures et ensuite concentrer sous vide. Reprendre le résidu dans l'eau et extraire au dichlorométhane. Purifier l'huile obtenue par chromatographie sur colonne de silice de 70-230 mesh en éluant avec un mélange de dichlorométhane et de méthanol (99:1 V/V)
Rendement : 38%
Spectre de résonance magnétique nucléaire du proton (200 MHz, solvant $CDCl_3$) : 1,8-2,1 ppm, m,2H; 1,2-1,4 ppm,t,3H; 2,3 ppm,s,3H; 2,05 ppm,t,2H; 2,2-2,6 ppm,m,8H; 3,55 ppm,t,1H; 4,1-4,3 ppm,q,2H; 6,8-7,2 ppm m 2H.

STADE C

Mélanger 0,01 mole de chlorhydrate d'amino-4 carbamoyl-5 imidazole, 0,011 mole de l'ester préparé dans l'étape précédente et 10,5 g d'acide phosphorique. Porter à 80°C pendant 30 minutes.

Hydrolyser par de la glace et neutraliser avec de la soude concentrée pour obtenir la précipitation de la {[[bis(fluoro-2 phényl) méthylène]-4 pipéridino]-2 éthyl}-4 carbamoyl-8 hydroxy-4 méthyl-2 imidazo [1,5-a] pyrimidine. Salifier ensuite avec de l'éthanol chlorhydrique.
Rendement : 30%
Point de fusion :>260°C

**EXEMPLE 2**

**Dichlorhydrate de la {[[(fluoro-4 phényl)phényl méthylène]-4 pipéridino]-2 éthyl}-2 hexahydro-1,3,4,6,11,11a-2H pyrazino [1,2-b] isoquinoléine dione-1,3**

Porter à reflux un mélange contenant 0,0247 mole de (chloro-2 éthyl)-2 hexahydro-1,3,4,6,11,11a-2H pyrazino [1,2-b] isoquinoléine, 0,0235 mole de [(fluoro-4 phényl)phényl méthylène]-4 pipéridine dione-1,3, 5 g de carbonate de sodium et 0,5 g d'iodure de sodium dans 200 ml de méthyléthylcétone, pendant 24 heures. Après évaporation du solvant, reprendre le résidu dans l'eau et extraire au benzène. Sécher la phase organique sur sulfate de sodium anhydre et concentrer sous vide. Purifier l'huile obtenue par chromatographie sur colonne contenant 150 g de silice (70-230 mesh). Eluer à l'aide d'un mélange de dichlorométhane et d'éthanol (99:1 V/V).

L'huile purifiée est transformée en dichlorhydrate dans l'éthanol chlorhydrique.
Rendement : 37%
Point de fusion : 150°C

**EXEMPLE 3**

**Chlorhydrate de la carbamoyl-8 {[[(fluoro-4 phényl) phényl méthylène]-4 pipéridino]-2 éthyl}-3 hydroxy-4 méthyl-2 imidazo [1,5-a] pyrimidine**

STADE A

Carbamoyl-8 hydroxy-4 hydroxyéthyl-3 méthyl-2 imidazo [1,5-a] pyrimidine

Dans un tricol introduire successivement 3 g de chlorhydrate d'amino-4 carbamoyl-5 imidazole, 1,51 g d'acétate de sodium, 1,69 g d'éthanol, 7 g d'acétyl-3 tétrahydrofurannone-2 et 45 ml de toluène. Chauffer 90 h à reflux. Après refroidissement, reprendre le précipité formé par l'éthanol bouillant. Filtrer et reprendre le résidu dans l'eau bouillante, filtrer, laver à l'éthanol le résidu et ensuite sécher.
Rendement : 62%
Spectre de résonance magnétique nucléaire du proton (400 MHz, solvant DMSO-$d_6$) : 2,5 ppm,s,3H; 2,6 ppm,t,2H; 3,5 ppm,q,2H; 4,6 ppm,t,1H; 7,1-7,4 ppm,m,2H; 8,1 ppm, s,1H; 11,4 ppm,m,1H

STADE B

A température ambiante, ajouter par portions 0,0061 mole de composé obtenu au stade précédent à une suspension de 0,012 mole d'hydrure de sodium dans 80 ml de diméthylformamide. Ajouter à la solution obtenue une solution contenant 0,012 mole d'iodure de phénylméthylamino triphényl phosphonium (H. Zimmer, et F. Singh, J.Org.Chem.,(1963),28,p483) et 0,015 mole de [(fluoro-4 phényl) phényl méthylène]-4 pipéridine dans 40 ml de diméthylformamide. Porter à 80 °C durant 40 heures, évaporer ensuite sous vide le diméthylformamide et reprendre le résidu dans l'eau. Extraire au chloroforme. L'huile obtenue après concentration est purifiée par chromatographie sur silice (70-230 mesh) en utilisant comme éluant un mélange de dichlorométhane méthanol et d'ammoniaque (90:10:0,2 V/V/V/).

Le chlorhydrate de la carbamoyle-8 {[[(fluoro-4 phényl) phényl méthylène]-4 pipéridino]-2 éthyl}-3 hydroxy-4 méthyl-2 imidazo [1,5-a] pyrimidine est obtenu après addition à la base purifiée d'éthanol chlorhydrique.

**EXEMPLE 4**

**Chlorhydrate de la cyano-8 {[[(fluoro-4 phényl) phényl méthylène]-4 pipéridino]-2 éthyl}-3 hydroxy-4 méthyl-2 imidazo [1,5-a] pyrimidine**

STADE A

Chloroéthyl-3 cyano-8 hydroxy-4 méthyl-2 imidazo [1,5-a] pyrimidine

Porter à 85 °C pendant une heure et 30 mn 0,17 mole du composé obtenu au Stade A de l'exemple 3 en solution dans 200 ml d'oxychlorure de phosphore. Eliminer ensuite ce dernier par évaporation sous vide. Ajouter 100 ml d'eau et ajuster le pH à 7 à l'aide du bicarbonate de sodium pour cristalliser le composé attendu. Filtrer.
Rendement : 88%
Point de fusion : 225 °C
Spectre de résonance magnétique nucléaire du proton (60 MHz, solvant DMSO-$d_6$) : 2,45 ppm,s,3H; 2,9 ppm,t,2H; 3,7 ppm,t,2H; 8,15 ppm,s,1H; 13-13,4 ppm,1H échangeable.

STADE B

Porter à reflux pendant 8 heures un mélange contenant 0,0046 mole de [(fluoro-4 phényl) phényl méthylène]-4 pipéridine, 0,0055 mole du composé obtenu au Stade A, 0,028 mole de carbonate de sodium et 0,1 g d'iodure de potassium dans 100 ml de méthyl-4 pentanone-2. Concentrer sous vide. Reprendre le résidu dans l'eau et extraire au dichlorométhane. Après évaporation du solvant, le produit est solubilisé dans un mélange d'éther éthylique et d'acétone et transformé en chlorhydrate après addition d'acide chlorhydrique. Le sel est recristallisé dans l'acétone.
Rendement : 45%
Point de fusion : 213-215 °C

**EXEMPLE 5**

**Chlorhydrate de la (fluoro-2 benzyl)-4 {[[(fluoro-4 phényl) phényl méthylène]-4 pipéridino]-3 propyl}-1 pipérazine dione-2,6**

A une suspension de 0,0112 mole d'hydrure de sodium dans 40 ml de diméthylformamide ajouter à 20 °C 0,0112 mole de (fluoro-2 benzyl)-4 pipérazine dione-2,6. Porter 45 minutes à 60 °C puis à 20 °C ajouter 0,013 mole de (chloro-3 propyl)-1 [(fluoro-4 phényl) phényl méthylène]-4 pipéridine. Après 15 heures d'agitation à 20 °C, évaporer le solvant sous vide. Reprendre le résidu dans l'eau et extraire au dichlorométhane. Purifier l'huile obtenue par chromatographie sur 520 g de silice (230-400 mesh), en éluant avec un mélange de dichlorométhane et d'éthanol (98:2 V/V).

Le chlorhydrate de la (fluoro-2 benzyl)-4 {[[(fluoro-4 phényl) phényl méthylène]-4 pipéridino]-3 propyl}-1 pipérazine dione-2,6 est obtenu après addition d'éthanol chlorhydrique et recristallisation dans un mélange d'acétone et d'éther éthylique.
Rendement : 26%

Point de fusion 199 °C

**EXEMPLE 6**

**Chlorhydrate de la {[(fluorènylidényl-9)-4 pipéridino]-3 propyl}-1 (fluoro-2 benzyl)-4 pipérazine dione-2,6**

STADE A

(Hydroxy-9 fluorènyl-9)-4 méthyl-1 pipéridine

A 20 °C, ajouter une solution de 0,412 mole de fluorènone dans 300 ml de tétrahydrofuranne à une solution de 0,659 mole de chlorure de (méthyl-1 pipéridyl-4) magnésium dans 600 ml de tétrahydrofuranne. Laisser agiter pendant une nuit, puis, à froid, hydrolyser avec une solution de chlorure d'ammonium. Concentrer le milieu réactionnel et reprendre le résidu dans un litre d'eau. Extraire au chloroforme. Sécher la phase organique sur sulfate de sodium anhydre et ensuite concentrer. Après cristallisation du produit attendu, laver à l'éther éthylique et filtrer.
Rendement : 50%
Point de fusion : 218 °C
Spectre de résonance magnétique nucléaire du proton (60 MHz, solvant $CDCl_3$) : 1,0-3,0 ppm, m,9H; 2,1 ppm,s,3H; 4,7-5,8 ppm, 1H échangeable; 7,2-8,0 ppm,m,8H.

STADE B

(Fluorènylidènyl-9)-4 méthyl-1 pipéridine

Porter à reflux pendant 12 heures un mélange contenant 0,258 mole de l'alcool obtenu au stade précédent, 740 ml d'acide acétique glacial et 220 ml d'acide chlorhydrique concentré. Concentrer ensuite le milieu réactionnel sous vide, neutraliser à l'hydroxyde de sodium 10N, extraire au dichlorométhane et évaporer le solvant. Le produit cristallise. Reprendre les cristaux à l'éther isopropylique.
Rendement : 47%
Point de fusion : 115 °C
Spectre de résonance magnétique nucléaire du proton (60 MHz, solvant $CDCl_3$) : 2,3 ppm,s,3H; 3,1-3,4 ppm,t 4H; 3,4 - 3,7 ppm,t,4H; 7,2-7,6 ppm,m,4H; 7,6-8,1 ppm,m,4H.

STADE C

Ethoxycarbonyl-1 (fluorènylidènyl-9)-4 pipéridine

Dissoudre 0,169 mole du composé obtenu au Stade B dans un litre de toluène anhydre, et ajouter 0,676 mole de chloroformiate d'éthyle et porter pendant 3 heures au reflux. Concentrer le solvant sous vide. Reprendre le produit cristallisé à l'éther isopropylique.
Rendement : 70%
Point de fusion : 124 °C
Spectre de résonance magnétique nucléaire du proton (60 MHz, solvant $CDCl_3$) : 1,2-1,5 ppm,t,3H; 3,2-3,6 ppm,t,4H; 3,6-4,0 ppm,t,4H; 4,1-4,5 ppm,q,2H; 7,3-7,6 ppm,m,4H; 7,6-8,1 ppm,m,4H.

STADE D

chlorhydrate de (fluorènylidènyl-9)-4 pipéridine

Porter pendant une heure à 60 °C un mélange de 0,01 mole du composé obtenu au stade précédent, 0,012 mole d'iodotriméthylsilane dans 4,2 ml de chloroforme. Filtrer ensuite le précipité obtenu sous argon, laver au chloroforme et le dissoudre dans 50 ml de méthanol anhydre. Ajouter 0,015 mole de sodium et évaporer le méthanol. Reprendre le résidu dans l'éther éthylique et du dichlorométhane. Filtrer et ajouter au filtrat du méthanol chlorhydrique pour obtenir le produit attendu sous forme de précipité blanc.
Rendement : 85%
Point de fusion : >260 °C

STADE E

Porter à reflux un mélange de 0,6 g de carbonate de potassium, 0,0044 mole du composé préparé au Stade D et 0,0044 mole de (chloro-3 propyl)-1 (fluoro-2 benzyl)-4 pipérazine dione-2,6 dans 40 ml de méthyl-4 pentanone-2 pendant trois heures. Ensuite concentrer le tout, reprendre dans 50 ml d'eau, extraire au dichlorométhane, sécher et concentrer. Purifier l'huile obtenue par chromatographie sur colonne contenant 100 g de silice (70-230 mesh) en utilisant comme éluant un mélange de dichlorométhane et d'éthanol (99:1 V/V). Former ensuite le chlorhydrate de la {[(fluorènylidènyl-9)-4 pipéridino]-3 propyl}-1 (fluoro-2 benzyl)-4 pipérazine dione-2,6 à l'aide de l'éthanol chlorhydrique.
Rendement : 42%
Point de fusion : 206 °C

**EXEMPLE 7**

**Dichlorhydrate de la {[[bis(fluoro-4 phényl)méthylène]-4 pipéridino]-3 propyl}-1 (pyrimidinyl-2)-4 pipérazine**

Porter à reflux pendant 48 heures un mélange de 0,89 g de (chloro-3 propyl)-1 (pyrimidinyl-2)-4 pipérazine de 0,80 g de [bis(fluoro-4 phényl) méthylène]-4 pipéridine, 0,51 g de carbonate de potassium dans 30 ml de méthyl-4 pentanone-2 en présence de traces d'iodure de potassium. Evaporer le solvant et reprendre le résidu dans le dichlorométhane. Laver la solution à l'eau. Concentrer. Le résidu obtenu est purifié sur colonne de silice en utilisant comme éluant un mélange de dichlorométhane et de méthanol (80:20 V/V).

On obtient 1,1 g d'huile qui est transformé en dichlorhydrate dans l'éthanol chlorhydrique.
Rendement : 69%
Point de fusion : 204 °C

**EXEMPLE 8**

**Dichlorhydrate de la {[[bis(fluoro-4 phényl)-2,2 vinylène]-3 pyrrolidinyl-1]-3 propyl}-1 (fluoro-2 benzyl)-4 pipérazine dione-2,6**

STADE A

(Benzyl-1 pyrrolidinyl-3)-2 acétate d'éthyle

A 0,299 mole de (benzyl-1 pyrrolidinyl-3) acétonitrile (Chem.Pharm.Bull.,(1977),25,(8),p.1911-1922) dans 150 ml d'éthanol à 95%, ajouter goutte à goutte 120 g d'acide sulfurique à 98%. Porter ensuite le mélange à reflux pendant 6 heures, refroidir l'ensemble à 10 °C et ajouter 600 g de glace. Neutraliser avec du carbonate de sodium, extraire à l'éther éthylique, sécher sur sulfate de sodium et concentrer. Distiller ensuite sous vide pour obtenir l'ester attendu.
Rendement : 73%
Point d'ébullition : 115-125 °C sous 0,02 mmHg
Spectre de résonance magnétique nuléaire du proton (200 MHz, solvant $CDCl_3$) : 1,1-1,3 ppm,t,3H; 1,3-1,5 ppm,m,1H; 1,9-2,2 ppm,m + m,1 + 1H; 2,4 ppm,d,2H; 2,4-2,7 ppm,m,3H; 2,7-2,9 ppm,d,1H; 3,6 ppm,s,2H; 4-4,2 ppm,q,2H; 7,1-7,4 ppm,m,5H.

STADE B

Benzyl-1 [bis(fluoro-4 phényl)-2,2 hydroxy-2 éthyl]-3 pyrrolidine

A 0,875 mole de magnésium dans 60 ml de tétrahydrofuranne, ajouter une solution de 0,946 mole de bromo-4 fluorobenzène dans 500 ml de tétrahydrofuranne. Porter l'ensemble 3 heures à reflux. Refroidir à 10 °C et additionner une solution de 0,218 mole du composé obtenu au stade précédent dans 150 ml de tétrahydrofuranne. Porter 20 heures à reflux, puis hydrolyser à froid avec une solution de chlorure d'ammonium. Diluer avec un litre d'eau et extraire au dichlorométhane. Ajouter à la phase organique un excès d'éthanol chlorhydrique, puis un litre d'éther éthylique. Filtrer et relarguer la base pour obtenir l'alcool attendu.

Rendement : 77%
Spectre de résonance magnétique nucléaire du proton (60 MHz, solvant $CDCl_3$) : 1,0-3,1 ppm,m,9H; 3,5 ppm,s,2H; 3,4-3,9 ppm,1H échangeable; 6,6-7,7 ppm,m,8H; 7,3 ppm,s,5H.

STADE C

Benzyl-1 [bis(fluoro-4 phényl)-2,2 vinylène]-3 pyrrolidine

Porter à reflux pendant 4 heures un mélange de 0,169 mole du composé obtenu au Stade B, de 460 ml d'acide acétique et de 145 ml d'acide chlorhydrique à 35%. Concentrer ensuite l'acide acétique sous vide, ajouter au résidu 400 ml d'eau, 400 ml de dichlorométhane et neutraliser l'ensemble avec de l'hydroxyde de sodium. La concentration de la phase organique donne 60 g d'une huile qui est purifiée par chromatographie sur colonne en utilisant 800 g de silice (70-230 mesh) et comme éluant un mélange de dichlorométhane de méthanol et d'ammoniaque (99:1:0,1 V/V/V). On obtient le produit attendu pur.
Rendement : 80%
Spectre de résonance magnétique nucléaire du proton (200 MHz, solvant $CDCl_3$) : 1,5-1,8 ppm,m,2H; 1,8-2,1 ppm,m,1H; 2,3 ppm,d,1H; 2,4-3,0 ppm,m,3H; 3,5-3,7 ppm,d,2H; 5,95 ppm,d,1H; 6,7-7,4 ppm,m,13H.

STADE D

[bis(fluoro-4 phényl)-2,2 vinylène]-3 ethoxycarbonyl-1 pyrrolidine

Mélanger 0,136 mole du composé obtenu au Stade C avec 0,272 mole de chloroformiate d'éthyle et 600 ml de toluène. Chauffer l'ensemble 4 heures à 100 °C, puis refroidir à 15 °C. Laver la phase organique avec l'acide chlorhydrique 1N, la sécher sur sulfate de sodium anhydre et concentrer sous vide. L'huile obtenue est utilisée sans purification dans l'étape suivante.

STADE E

Bis(fluoro-4 phényl)-1,1 (pyrrolidinyl-3)-2 vinyle

Porter pendant trois heures au reflux un mélange de 0,014 mole de l'huile obtenue au Stade D et 25 ml d'acide bromhydrique à 48%, puis refroidir, laver la phase aqueuse à l'éther éthylique et neutraliser avec de l'hydroxyde de sodium 2N. Extraire la phase aqueuse à l'éther éthylique et concentrer. L'huile obtenue est purifiée par chromatographie sur colonne contenant 50 g de silice (70-230 mesh) et en utilisant comme éluant un mélange de dichlorométhane et de méthanol (98:2 V/V).
Rendement : 48%
Spectre de résonance magnétique nucléaire du proton (60 MHz, solvant DMSO-$d_6$) : 1,8-2,2 ppm,m,2H; 2,6-3,5 ppm,m + m,1H + 4H; 6,2 ppm,d,1H; 7,2-7,6 ppm,m,8H; 9-10 ppm,2H échangeables.

STADE F

Porter à reflux un mélange de 0,017 mole de (chloro-3 propyl)-1 (fluoro-2 benzyl)-4 pipérazine dione-2,6, de 3,4 g de carbonate de sodium, de 0,015 mole du composé obtenu au Stade E et de 100 ml de méthyl-4 pentanone-2 pendant 8 heures. Concentrer le mélange réactionnel, ajouter 100 ml d'eau distillée et extraire l'ensemble avec 300 ml de dichlorométhane. L'huile obtenue après concentration du solvant est purifiée sur colonne chromatographique contenant 350 g de silice (70-230 mesh) en utilisant comme éluant un mélange de dichlorométhane et de méthanol (99:1 V/V).
Rendement : 30%

Pour former le dichlorhydrate de la {[[bis(fluoro-4 phényl)-2,2 vinylène]-3 pyrrolidinyl-1]-3 propyl}-1 (fluoro-2 benzyl)-4 pipérazine dione-2,6, dissoudre la base dans un mélange d'acétone et d'éther éthylique et ajouter deux équivalents d'éthanol chlorhydrique. Concentrer, précipiter à l'aide de l'éther éthylique, filtrer et sécher sous vide. Point de fusion : 160 °C

**EXEMPLE 9**

**Chlorhydrate de la carbamoyl-8 {[[(fluoro-4 phényl)phényl méthylène]-4 pipéridino]-3 propyl}-3 hydroxy-4 méthyl-2 imidazo [1,5-a] pyrimidine**

STADE A

Acétyl-2 {[(fluoro-4 phényl)phényl méthylène]-4 pipéridino}-5 pentanoate d'éthyle

Porter à reflux 0,019 mole de la [(fluoro-4 phényl)phényl méthylène]-4 pipéridine, 0,019 mole d'acétyl-3 chloro-5 pentanoate d'éthyle bloqué sous forme cyclique (Chem.Ber.(1967),100,p.1675-1679), et 2,03 g de carbonate de sodium dans 80 ml de butanone-2. Après évaporation du solvant, reprendre le résidu dans l'eau et extraire au dichlorométhane. Ensuite procéder à la déprotection, selon la méthode décrite dans Chem.Ber.(1967),100,p.1675-1679.
Rendement : 40%
Spectre de résonance magnétique nucléaire du proton (60 MHz, solvant $CDCl_3$): 1,1-1,4 ppm,t,3H; 1,2-2,1 ppm,m,4H; 2,25 ppm,s,3H; 2,0-3,0 ppm,m,10H; 3,3-3,7 ppm,t,1H; 4,0-4,5 ppm,q,2H; 7,0-7,6 ppm,m,9H.

STADE B

Le chlorhydrate de la carbamoyl-8 {[[(fluoro-4 phényl)phényl méthylène]-4 pipéridino]-3 propyl}-3 hydroxy-4 méthyl-2 imidazo [1,5-a] pyrimidine est préparé en condensant l'amino-4 carbamoyl-5 imidazole avec le composé obtenu au stade précédent selon le procédé décrit dans l'exemple 1, Stade C.
Rendement : 30%
Point de fusion : 245 ° C

**EXEMPLE 10**

**Dichlorhydrate de {[[(fluoro-4 phényl)phényl méthylène]-4 pipéridino]-3 propyl}-1 (pyridyl-2 méthyl)-4 pipérazine dione-2,6**

Ce composé a été préparé à partir de la [(fluoro-4 phényl)phényl méthylène]-4 pipéridine et de la (chloro-3 propyl)-1 (pyridyl-2 méthyl)-4 pipérazine dione-2,6 selon le procédé décrit dans l'exemple 6, Stade E.
Rendement : 35%
Point de fusion : 145 ° C

**EXEMPLE 11**

**Chlorhydrate de la {[[(bis(fluoro-4 phényl) méthylène]-4 pipéridinol-2 éthyl}-3 cyano-8 hydroxy-4 méthyl-2 imidazo [1,5-a] pyrimidine**

Ce composé a été préparé à partir de la chloroéthyl-3 cyano-8 hydroxy-4 méthyl-2 imidazo [1,5-a] pyrimidine et de la [bis(fluoro-4 phényl) méthylène]-4 pipéridine selon le procédé décrit dans l'exemple 4, Stade B.
Rendement : 47%
Point de fusion : 287 ° C

**EXEMPLE 12**

**Trichlorhydrate de la {[[(fluoro-4 phényl)phényl méthylène]-4 pipéridino]-2 éthyl}-1 (pyridyl-2 méthyl)-4 pipérazine dione-2,6**

Ce composé a été préparé à partir de la [(fluoro-4 phényl)phényl méthylène]-4 pipéridine et de la (chloro-2 éthyl)-1 (pyridyl-2 méthyl)-4 pipérazine dione-2,6 selon le procédé décrit dans l'exemple 6, Stade E.
Rendement : 41%
Point de fusion : 171 ° C

**EXEMPLE 13**

**Chlorhydrate de la carbamoyl-8 hydroxy-4 {[[(méthyl-4 phényl)phényl méthylène]-4 pipéridino]-2 éthyl}-3 méthyl-2 imidazo [1,5-a] pyrimidine**

Ce composé a été préparé à partir de l'acétyl-2 {[(méthyl-4 phényl) phényl méthylène]-4 pipéridyl-1}-4 butyrate d'éthyle et de chlorhydrate d'amino-4 carbamoyl-5 imidazole, selon le procédé décrit dans l'exemple 1.
Rendement : 38%
Point de fusion : 260 °C

**EXEMPLE 14**

**Dichlorhydrate de la {[[(bis(fluoro-4 phényl)méthylène]-4 pipéridino]-3 propyl}-1 (isoquinolyl-1)-4 pipérazine**

En procédant comme dans l'exemple 7 mais en remplaçant la (pyrimidinyl-2)-1 pipérazine par l'-(isoquinolyl-1)-1 pipérazine, on obtient le produit du titre.

**EXEMPLE 15**

**Dichlorhydrate de la {[[(bis(fluoro-4 phényl)méthylène]-4 pipéridino]-3 propyl}-1-(quinolyl-2)-4 pipérazine**

En procédant comme dans l'exemple 7 mais en remplaçant la (pyrimidinyl-2)-1 pipérazine par la (quinolyl-2)-1 pipérazine, on obtient le produit du titre.

**EXEMPLE 16**

**Dichlorhydrate de la {[[(bis(fluoro-4 phényl)méthylène]-4 pipéridino]-3 propyl}-1 (trifluorométhyl-2 benzyl)-4 pipérazine**

En procédant comme dans l'exemple 7 mais en remplaçant la (pyrimidinyl-2)-1 pipérazine par la (trifluorométhyl-2 benzyl)-1 pipérazine, on obtient le produit du titre.

**EXEMPLE 17**

**Dichlorhydrate de la {[[(bis(fluoro-4 phényl)méthylène]-4 pipéridino]-3 propyl}-1 (thiazolyl-2)-4 pipérazine**

En procédant comme dans l'exemple 7 mais en remplaçant la (pyrimidinyl-2)-1 pipérazine par la (thiazolyl-2)-1 pipérazine, on obtient le produit du titre.

**EXEMPLE 18**

**Dichlorhydrate de la {[[(bis(fluoro-4 phényl)méthylène]-4 pipéridino]-3 propyl}-1 (méthyl-4 thiazolyl-2)-4 pipérazine**

En procédant comme dans l'exemple 7 mais en remplaçant la (pyrimidinyl-2)-1 pipérazine par la (méthyl-4 thiazolyl-2)-1 pipérazine, on obtient le produit du titre.

**EXEMPLE 19**

**Dichlorhydrate de la {[[(bis(fluoro-4 phényl)méthylène]-4 pipéridino]-3 propyl}-1 (phényl-4 thiazolyl-2)-4 pipérazine**

En procédant comme dans l'exemple 7 mais en remplaçant la (pyrimidinyl-2)-1 pipérazine par la (phényl-4 thiazolyl-2)-1 pipérazine, on obtient le produit du titre.

**EXEMPLE 20**

**Dichlorhydrate de la {[[(bis(fluoro-4 phényl)méthylène]-4 pipéridino]-3 propyl}-1 (diméthyl-4,5 thiazolyl-2)-4 pipérazine**

En procédant comme dans l'exemple 7 mais en remplaçant la (pyrimidinyl-2)-1 pipérazine par la (diméthyl-4,5 thiazolyl-2)-1 pipérazine, on obtient le produit du titre.

**EXEMPLE 21**

**Dichlorhydrate de la {[[(bis(fluoro-4 phényl)méthylène]-4 pipéridino]-3 propyl}-1 (benzothiazolyl-2)-4 pipérazine**

En procédant comme dans l'exemple 7 mais en remplaçant la (pyrimidinyl-2)-1 pipérazine par la (benzothiazolyl-2)-1 pipérazine, on obtient le produit du titre.

**EXEMPLE 22**

**Chlorhydrate de la {[[bis (fluoro-4 phényl) méthylène]-4 perhydroazépino]-2 éthyl}-3 carbamoyl-8 hydroxy-4 méthyl-2 imidazo [1,5-a] pyrimidine**

STADE A

N,N-diméthyl amino-4 butyronitrile

A une solution chauffée à 80°C de 400 g de chloro-4 butyronitrile dans 1200 ml d'éthanol pur, ajouter 420 g de diméthylamine. Après 12 heures à reflux, concentrer le solvant, y ajouter ensuite 3 litres d'éther éthylique, et éliminer le précipité formé. Concentrer le filtrat et le distiller sous 12 mm Hg.

Spectre de résonance magnétique nucléaire du proton : (200 MHz, solvant $CDCl_3$) : 1,75 ppm,q,2H; 2,25 ppm,s,6H; 2,3-2,5 ppm,t + t,2H + 2H

STADE B

(N,N-diméthylamino-2 éthyl)-2 chloro-5 pentanenitrile

Ajouter une solution de 0,800 mole du produit obtenu au stade précédent à une solution refroidie à -85°C de 0,800 mole de diisopropyl-amidure de lithium dans 600 ml de tétrahydrofuranne. Laisser 20 minutes à -85°C, puis y additionner 0,800 mole de chloro-3 iodo-1 propane pur en 5 minutes. Laisser agiter pendant une heure puis hydrolyser à -80°C avec 500 ml d'acide acétique à 3%. Concentrer sous vide, reprendre dans 250 ml d'eau, extraire au dichlorométhane, concentrer cet extrait.
Rendement : 90%

Spectre de résonance magnétique nucléaire du proton : (200 MHz, solvant $CDCl_3$) : 1,6-2,1 ppm,m,6H; 2,2 ppm,s,6H; 2,3-2,6 ppm,m,2H; 2,75 ppm,m,1H; 3,6 ppm,t,2H

STADE C

Cyano-4 méthyl-1 perhydroazépine

Porter 12 heures à 120°C un mélange de 140 g du produit précédent et de 850 ml de nitrobenzène, puis refroidir à 15°C et y ajouter 2 litres d'éther éthylique. Filtrer le précipité obtenu et le rincer à l'éther. Mélanger ensuite le produit obtenu avec un litre de décanol et porter l'ensemble à reflux pendant deux heures. Refroidir ensuite, extraire quatre fois avec 500 ml d'acide chlorhydrique 1N, neutraliser cet extrait à la soude et l'extraire au dichlorométhane. Distiller l'huile sous vide.
Rendement : 70%

Spectre de résonance magnétique nucléaire du proton : (200 MHz, solvant $CDCl_3$) : 1,5-2,2 ppm,m, 6H; 2,35 ppm,s,3H; 2,5-3,0 ppm,m + m,1H + 4H

STADE D

(méthyl-1 perhydroazépinyl-4)-2 acétate d'éthyle

Ce composé a été préparé à partir de cyano-4 méthyl-1 perhydroazépine, composé obtenu au stade précédent, selon le procédé décrit au stade A de l'exemple 8.
Rendement : 90%
Point d'ébullition : 46-48 ° C sous 0,03 mmHg.

STADE E

Bis (fluoro-4 phényl)-1,1 (méthyl-1 perhydroazépinyl-4)-1 méthanol

Ce composé a été préparé à partir du produit obtenu au stade D et selon le procédé décrit au stade B de l'exemple 8.
Rendement : 85%
Point de fusion : 109 ° C.

STADE F

[bis (fluoro-4-phényl) méthylène]-4 méthyl-1 perhydroazépine

Ce composé a été préparé à partir du produit obtenu au stade précédent et selon le procédé décrit au stade C de l'exemple 8.
Rendement : 75%
Spectre de résonance magnétique nucléaire du proton : (200 MHz, solvant $CDCl_3$) : 1,8 ppm,m,2H; 2,3-2,5 ppm,m + s,2H + 3H; 2,5-2,7 ppm,m,6H; 6,9-7,15 ppm,m,8H.

STADE G

[bis (fluoro-4-phényl) méthylène]-4 éthoxy-carbonyl-1 perhydroazépine

Ce composé a été préparé à partir de la [bis (fluoro-4-phényl) méthylène]-4 méthyl-1 perhydroazépine et selon le procédé décrit au stade D de l'exemple 8.
Rendement : 95%

STADE H

[bis (fluoro-4-phényl) méthylène]-4 perhydroazépine

Ce composé a été préparé à partir du produit au stade précédent et selon le procédé décrit au stade E de l'exemple 8.
Rendement : 80%

STADE I

Chlorhydrate de la [bis (fluoro-4-phényl) méthylène]-4 chloroéthyl-1 perhydroazépine

Ce composé a été préparé à partir de la [bis (fluoro-4-phényl) méthylène]-4 perhydroazépine et selon le procédé décrit au stade A de l'exemple 1.

STADE J

Acètyl-2 {[bis (fluoro-4 phenyl) méthylène]-4 perhydroazépino}-4 butyrate d'éthyle

Ce composé a été préparé à partir du composé décrit au stade précédent et selon le procédé décrit au stade B de l'exemple 1.
Rendement : 38%

Spectre de résonance magnétique nucléaire du proton : (200 MHz, solvant $CDCl_3$) : 1,3 ppm,t,3H; 1,6-1,8 ppm,m,2H; 1,9-2,7 ppm,m + s + m, 10H + 3H + 2H; 3,55 ppm,t,1H; 4,15 ppm,q,2H; 6,8-7,15 ppm,m,8H.

STADE K

Le chlorhydrate de la {[[(bis (fluoro-4 phényl) méthylène]-4 perhydroazépino]-2 éthyl}-3 carbamoyl-8 hydroxy-4 méthyl-2 imidazo [1,5-a] pyrimidine a été obtenu à partir de l'ester préparé au stade précédent et selon le procédé décrit au stade C de l'exemple 1.
Rendement : 30%
Point de fusion : > 260 °C.

**EXEMPLE 23**

**Chlorhydrate de la {[[bis (fluoro-4 phényl)-2,2 vinylène]-3 pipéridino]-2 éthyl}-3 carbamoyl-8 hydroxy-4 méthyl-2 imidazo [1,5-a] pyrimidine**

STADE A

(Benzyl-1 pipéridyl-3)-2 acétate d' éthyle

A une solution refroidie à 5 °C de 34 g de (benzyl-1 pipéridyl-3) acétonitrile (J.Pharm.Sciences, Vol.55, N °5, mai 1966, p.535) dans 219 g d'éthanol anhydre et 350 ml d'éther éthylique anhydre, ajouter du chlorure d'hydrogène gazeux jusqu'à saturation. Porter alors à reflux en maintenant un léger barbottage de chlorure d'hydrogène. Concentrer, reprendre le résidu dans 150 ml d'eau, y ajouter 300 ml d'éther éthylique et neutraliser la solution à l'aide d'une solution saturée de carbonate de sodium. Décanter, extraire deux fois par 200 ml d'éther éthylique. Sécher la phase organique sur sulfate de sodium anhydre et concentrer. L'huile obtenue est chromatographiée sur 450 g de silice Merck 60 70/230 mesh, en utilisant comme éluant un mélange d'éther éthylique et d'hexane (50:50 V/V).
Rendement : 65%.
Spectre de résonance magnétique nucléaire du proton : (200 MHz, solvant $CDCl_3$) : 1-1,3 ppm,t,3H; 0,7-3 ppm,m,11H; 3,45 ppm,s,2H; 3,9-4,3 pm,q,2H; 7,3 ppm,s,5H.

STADE B

Chlorhydrate de benzyl-1 [bis (fluoro-4 phényl)-2,2 hydroxy-2 éthyl]-3 pipéridine

A 7,41 g de magnésium en suspension dans 20 ml de tétrahydrofuranne, ajouter sous azote une solution contenant 58 g de bromo-1 fluoro-4 benzène dans 100 ml de tétrahydrofuranne. Porter le mélange 2 heures à reflux, puis refroidir à 10 °C et y ajouter une solution de 20 g du composé obtenu dans le stade précédent en solution dans 150 ml de tétrahydrofuranne. Porter 10 heures à reflux puis hydrolyser avec 80 ml d'une solution saturée de'chlorure d'ammonium à 0 °C. Extraire 3 fois avec 200 ml d'éther éthylique, sécher les phases organiques sur sulfate de sodium anhydre et concentrer. Précipiter le monochlorhydrate formé dans l'acétone et filtrer.
Rendement : 80%
Spectre de résonance magnétique nucléaire du proton : (200 MHz, solvant $CDCl_3$ + DMSO-$d_6$) : 1,0-4,2 ppm,m + m + m,2H + 4H + 7H; 6,7-7,7 ppm,m,13H.

STADE C

Benzyl-1 [bis (fluoro-4 phényl)-2,2 vinylène]-3 pipéridine

Porter à reflux pendant 3 heures un mélange de 21 g du produit obtenu au stade précédent, 30 ml d'acide chlorhydrique concentré et 100 ml d'acide acétique glacial. Concentrer, reprendre le résidu dans de la soude 1N et extraire au dichlorométhane. Sécher cette phase organique sur sulfate de sodium anhydre et concentrer. Recristalliser dans l'éthanol. Rendement : 70%
Spectre de résonance magnétique nucléaire du proton : (200 MHz, solvant $CDCl_3$) : 1,5-3,5 ppm,m,9H; 4-4,2 ppm,m,2H; 5,5 ppm,d,1H; 6,8-7,6 ppm,m,13H.

STADE D

Bis (fluoro-4 phényl)-1,1 (pipéridyl-3)-2 vinyle

Ce composé a été préparé à partir du produit obtenu au stade C et selon le procédé décrit dans l'exemple 8 stades D et E.
Rendement : 77%

Spectre de résonance magnétique nucléaire du proton : (200 MHz, solvant DMSO-$d_6$) : 1,3-1,9 ppm,m,4H; 2,3-2,6 ppm,m,1H; 2,6-2,9 ppm,m,2H; 3-3,2 ppm,m,2H; 5,9 ppm,d,1H; 7-7,5 ppm,m,8H.

STADE E

Acétyl-2 {[bis (fluoro-4 phényl)-2,2 vinylène]-3 pipéridino}-4 butyrate d'ethyle

Ce composé a été préparé à partir du bis (fluoro-4 phényl)-1,1 (pipéridyl-3)-2 vinyle selon les procédés décrits dans les stades A et B de l'exemple 1.
Rendement : 35%

STADE H

Le chlorhydrate de la {[[bis (fluoro-4 phényl)-2,2 vinylène]-3 pipéridino]-2 éthyl}-3 carbamoyl-8 hydroxy-4 méthyl-2 imidazo [1,5-a] pyrimidine a été obtenu à partir du composé décrit au stade précédent selon le procédé décrit dans le stade C de l'exemple 1.
Rendement : 25%
Point de fusion : > 260 ° C.

**EXEMPLE 24**

**Chlorhydrate de la {[[bis (fluoro-4 phényl)-2,2 vinylène]-4 pyrrolidino]-3 éthyl}-3 carbamoyl-8 hydroxy-4 méthyl-2 imidazo [1,5-a] pyrimidine**

Pour préparer ce composé, procéder selon le procédé décrit dans l'exemple 1 en remplaçant la [bis (fluoro-4 phényl) méthylène]-4 pipéridine par le composé obtenu dans le stade E de l'exemple 8.
Rendement : 30%
Point de fusion : ≃ 265 ° C.

**EXEMPLE 25**

**Chlorhydrate de la {[[bis (fluoro-4 phényl) méthylène]-4 perhydroazépinyl-1]-2 éthyl}-3 dioxo-2,4 tétrahydro-1,2,3,4 quinazoline**

Porter 12 heures à reflux un mélange de 7,5 g de (chloro-2 éthyl)-3 dioxo-2,4 tétrahydro-1,2,3,4 quinazoline, 10 g de [bis(fluoro-4 phényl) méthylène]-4 perhydroazépine, 3,4 g d'hydrogénocarbonate de sodium et 100 ml de toluène. Filtrer et concentrer sous vide. L'huile obtenue est purifiée par chromatographie sur 500 g de silice 70-230 mesh en utilisant comme éluant un mélange de dichlorométhane, de méthanol et d'ammoniaque (98,4:1,5:0,1 V/V).

Le chlorhydrate correspondant est précipité dans l'acétone.
Rendement : 60%
Point de fusion : > 260 ° C.

**EXEMPLE 26**

**Chlorhydrate de la {[[bis (fluoro-4 phényl) méthylène]-4 perhydroazépinyl-1]-2 éthyl}-3 chloro-7 dioxo-2,4 tétrahydro-1,2,3,4 quinazoline.**

En procédant comme il est décrit dans l'exemple 25 mais en remplaçant la (chloro-2 éthyl)-3 dioxo-2,4 tétrahydro-1,2,3,4 quinazoline par la (chloro-2 éthyl)-3 chloro-7 dioxo-2,4 tétrahydro-1,2,3,4 quinazoline, on obtient le produit du titre.

Point de fusion : 260 °C.

**EXEMPLE 27**

**Chlorhydrate de la {[[bis (fluoro-4 phényl) méthylène]-4 pipéridino]-2 éthyl}-2 dihydro-1,2 oxo-1 phtalazine**

STADE A

Dihydro-1,2 oxo-1 [(tétrahydropyrannyl-2) éthoxy]-2 phtalazine

Ajouter une solution de 41,5 g d'hydroxyde de potassium dans 53 ml d'eau à une solution de 61 g de dihydro-1,2 oxo-1 phtalazine dans 430 ml diméthylsulfoxyde. Après une heure, y additionner rapidement 130 g de (bromo-2 éthoxy)-2 tétrahydropyranne et laisser agiter à 20 °C pendant 48 heures. Etendre alors la solution obtenue dans 1200 ml d'eau, extraire au dichlorométhane, sécher sur sulfate de sodium anhydre et concentrer.
Rendement : 80%

Spectre de résonance magnétique nucléaire du proton : (200 MHz, solvant $CDCl_3$) : 1,3-2 ppm,m,6H; 3,3-4,8 ppm,m + m, 1H + 6H; 7,6-7,9 ppm,m,3H; 8,15 ppm,s,1H; 8,4 ppm,m,1H.

STADE B

(chloro-2 éthyl)-2 dihydro-1,2 oxo-1 phtalazine

Porter 12 heures à 50 °C un mélange de 90 g du produit obtenu au stade A, 600 ml d'acide acétique pur, 300 ml de tétrahydrofuranne et 150 ml d'eau. Concentrer sous vide et purifier l'huile obtenue par chromatographie sur 1400 g de silice 60 (70-230 mesh) en utilisant comme éluant un mélange de dichlorométhane, de méthanol et d'ammoniaque (99:1:1 V/V).

L'huile obtenue est mélangée avec 400 ml de chloroforme et 35 g de chlorure de thionyle, et chauffée 3 heures à reflux. Concentrer, et recristalliser le solide obtenu dans l'éther isopropylique.
Rendement : 45%

Spectre de résonance magnétique nucléaire du proton : (200 MHz, solvant $CDCl_3$) : 3,95 ppm,t,2H; 4,6 ppm,t,2H; 7,15-7,9 ppm,m,3H; 8,2 ppm,s,1H; 8,4 ppm,m,1H.

STADE C

Porter 24 heures à reflux un mélange de 6,5 g du composé obtenu au stade précédent, 8 g de [bis (fluoro-4 phényl) méthylène]-4 pipéridine, 7,1 g d'hydrogènocarbonate de sodium et 200 ml de toluène. Filtrer, concentrer, purifier l'huile obtenue par chromatographie sur 600 g de silice 60 (70-230 mesh) en utilisant comme éluant un mélange de dichlorométhane, de méthanol et d'ammoniaque (99:1:0,1 V/V).

Former ensuite le chlorhydrate du produit dans l'acétone à l'aide d'acide chlorhydrique.
Rendement : 40%
Point de fusion : 170-172 °C.

TABLEAU I

COMPOSES DE FORMULE GENERALE I

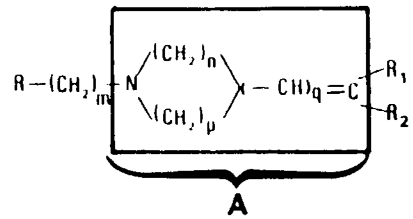

| EXEMPLE N° | R | m | A | R1 | R2 | SPECTRE R.M.N. (SOLVANT) |
|---|---|---|---|---|---|---|
| 1 | 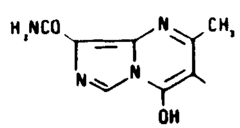 | 2 | 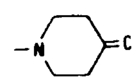 | 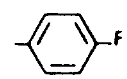 | 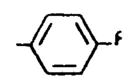 | RMN-$^1$H (DMSO-d6) sel<br>2,6 ppm,s,3H; 2,0-3,8 ppm,m, 12H; 6,9-7,7 ppm,m,8H; 8,2 ppm, s,1H. |
| 2 | 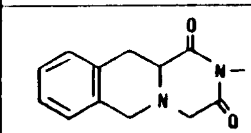 | 2 | 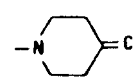 | 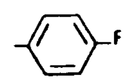 | 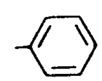 | RMN-$^1$H (DMSO-d6) sel<br>2,0-5,0 ppm,m,19H; 7,0-7,6 ppm,m,13H |
| 3 | 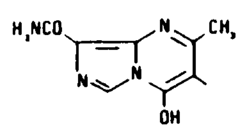 | 2 | 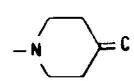 | 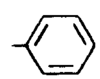 | 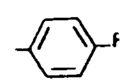 | RMN-$^1$H (DMSO-d6) sel<br>2,6 ppm,s,3H; 2,0-3,8 ppm, m,12H; 6,9-7,7 ppm,m,9H; 8,2 ppm,s,1H |

TABLEAU I

SUITE I

| EXEMPLE N° | R | m | A | R1 | R2 | SPECTRE R.M.N. (SOLVANT) |
|---|---|---|---|---|---|---|
| 4 | 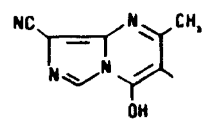 | 2 | 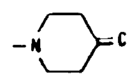 | 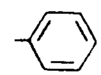 | 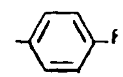 | RMN-$^1$H (DMSO-d6) sel 2,3-4,1 ppm,m,15H; 7-7,75 ppm,m,9H; 8,2 ppm,m,1H |
| 5 | 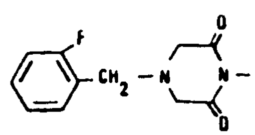 | 3 | 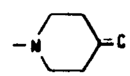 | 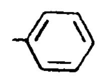 | 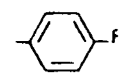 | RMN-$^1$H (DMSO-d6) sel 1,5-2,5 ppm,m,6H; 2,5-4 ppm, m,+s+s,8+2+2H; 6,8-7,5 ppm,m,13H; 10,5-11,5 ppm,m, 1H échangeable |
| 6 | 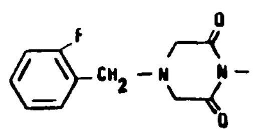 | 3 | 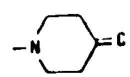 | 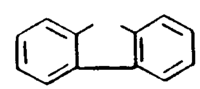 | | RMN-$^1$H (DMSO-d6) sel 1,6-2,4 ppm,m,2H; 2,6-4 ppm, t+s+s+m,2+2+4+10H; 6,8-8,1 ppm,m,12H; 11-12 ppm,1H échangeable |

TABLEAU I
SUITE II

| EXEMPLE N° | R | m | A | R1 | R2 | SPECTRE R.M.N. (SOLVANT) |
|---|---|---|---|---|---|---|
| 7 | 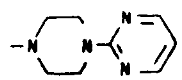 | 3 | 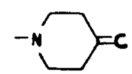 | 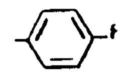 | 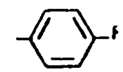 | RMN-$^1$H (DMSO-$d_6$) sel 2-2,8 ppm,m,2H; 2,8-4 ppm,m, 20H; 4,5-5 ppm, 1H échangeable; 6,7-7 ppm,t,1H; 7,1-7,5 ppm,m,8H; 8,5 ppm,d,2H; 11-12,2 ppm,1H échangeable |
| 8 | 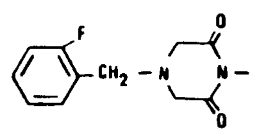 | 3 | 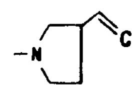 | 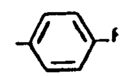 | 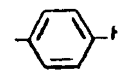 | RMN-$^1$H ($CDCl_3$) base 1,5-2,1 ppm,m,4H; 2,2-3,1 ppm,m,7H; 3,45 ppm,s,4H; 3,6-4,1 ppm,t+s,2+2H; 5,9-6,2 ppm,d,1H; 6,5-8 ppm,m,12H |
| 9 | 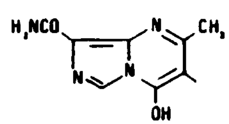 | 3 | 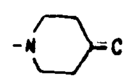 | 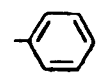 | 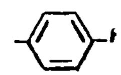 | RMN-$^1$H (DMSO-$d_6$) sel 1,7-2 ppm,m,2H; 2,3-2,7 ppm, s+m,3+2H; 2,7-3,2 ppm,m, 4H; 3,3-4 ppm,m,6H; 6,9-7,5 ppm,m,9H; 8,1 ppm,s,1H; 10,3-10,6 ppm,2H échangeables; 11,5 ppm,1H échangeable |

TABLEAU I
SUITE III

| EXEMPLE N° | R | m | A | $R_1$ | $R_2$ | SPECTRE R.M.N. (SOLVANT) |
|---|---|---|---|---|---|---|
| 10 | 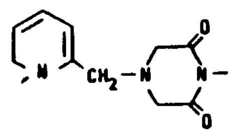 | 3 | 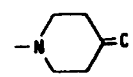 | 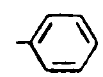 | 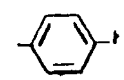 | RMN-$^1$H ($D_2O$) sel<br>1,5-4,0 ppm,s+m+m+m,4+2+10+2H; 4,1 ppm,s,2H; 6,5-7,3 ppm,m,9H; 7,7-8,1 ppm,m,2H; 8,3-8,7 ppm,m,2H |
| 11 | 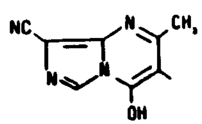 | 2 | 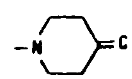 | 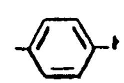 | 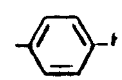 | RMN-$^{13}$C (DMSO-$d_6$) sel<br>17,2 ppm; 19,5 ppm; 27,8 ppm; 51,9 ppm; 53,4 ppm; 101,0 ppm; 111,5 ppm; 114,8 ppm; 115,1 ppm; 126,4 ppm; 130,4 ppm; 131,0 ppm; 135,5 ppm; 137,3 ppm; 137,7 ppm; 151,07 ppm; 155,5 ppm; 161,3 ppm |

TABLEAU I
SUITE IV

| EXEMPLE N° | R | m | A | R1 | R2 | SPECTRE R.M.N. (SOLVANT) |
|---|---|---|---|---|---|---|
| 12 | 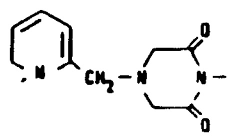 | 2 | 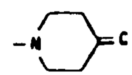 | 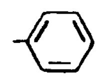 | 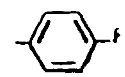 | RMN-$^1H$ (DMSO-d6) sel 2,4-3,9 ppm,m,6+4H; 3,9 ppm, s,4H; 3,9-4,3 ppm,m,2H; 4,4 ppm,s,2H; 7,1-7,6 ppm,m,9H; 8-9,1 ppm,m,4H; 10,5-11,6 ppm,3H échangeables |
| 13 | 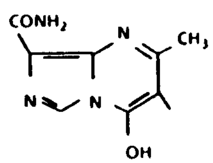 | 2 | 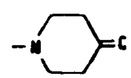 | 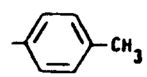 | 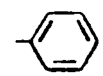 | RMN-$^{13}C$ (DMSO-d6) sel 17,3 ppm; 19,7 ppm; 20,5 ppm; 27,8 ppm; 52,2 ppm; 53,7 ppm; 101,1 ppm; 115,2 ppm; 123,4 ppm; 126,7 ppm; 128,2 ppm; 128,8 ppm; 128,9 ppm; 129,0 ppm; 132,6 ppm; 136,0 ppm; 137,9 ppm; 138,2 ppm; 141,3 ppm; 151,2 ppm; 155,8 ppm; 164,3 ppm |

TABLEAU I
SUITE V

| EXEMPLE N° | R | m | A | R1 | R2 | SPECTRE R.M.N. (SOLVANT) |
|---|---|---|---|---|---|---|
| 22 | 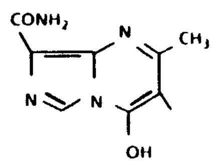 | 2 | 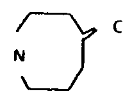 | 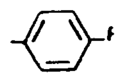 | 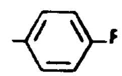 | RMN-$^{13}C$ (DMSO-$d_6$) sel 17,4 ppm; 19,9 ppm; 22,7 ppm; 28,5 ppm; 30,6 ppm; 52,5 ppm; 54,1 ppm; 54,2 ppm; 100,3 ppm; 114,9 ppm; 115,1 ppm; 115,2 ppm; 123,3 ppm; 130,6 ppm; 132,7 ppm; 135,2 ppm; 137,3 ppm; 138,2 ppm; 138,3 ppm; 151,1 ppm; 155,9 ppm; 159,7 ppm; 162,2 ppm; 164,3 ppm |

TABLEAU I
SUITE VI

| EXEMPLE N° | R | m | A | R1 | R2 | SPECTRE R.M.N. (SOLVANT) |
|---|---|---|---|---|---|---|
| 23 | 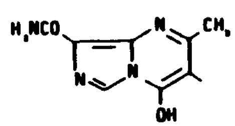 | 2 | 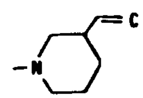 | 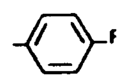 | 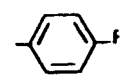 | RMN-$^{13}C$ (DMSO-$d_6$) sel 17,2 ppm, 19,3 ppm; 21,9 ppm; 28,2 ppm; 34,8 ppm; 51,2 ppm; 54,5 ppm; 54,6 ppm; 99,9 ppm; 115,5 ppm; 123,4 ppm; 128,8 ppm; 131,2 ppm; 132,5 pm; 134,9 ppm; 137,6 ppm; 141,0 ppm; 151,4 ppm; 155,9 ppm; 160,2 ppm; 162,8 ppm; 164,3 ppm |

TABLEAU I
SUITE VII

| EXEMPLE N° | R | m | A | R1 | R2 | SPECTRE R.M.N. (SOLVANT) |
|---|---|---|---|---|---|---|
| 24 | 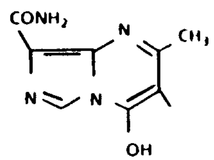 | 2 | 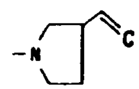 | 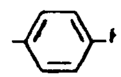 | 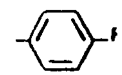 | **RMN-$^{13}$C (DMSO-$d_6$) sel**<br>17,2 ppm; 21,1 ppm; 29,8 ppm; 31,0 ppm; 52,2 ppm; 53,0 ppm; 57,0 ppm; 115,0 ppm; 115,4 ppm; 123,4 ppm; 128,6 ppm; 130,2 ppm; 132,5 ppm; 134,9 ppm; 137,6 ppm; 140,6 ppm; 151,7 ppm; 155,8 ppm; 159,8 ppm; 163,0 ppm; 164,3 ppm |
| 25 | 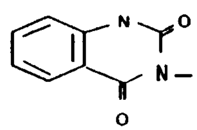 | 2 | 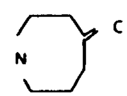 | 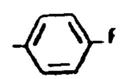 | 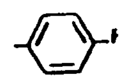 | **RMN-$^{1}$H (DMSO-$d_6$) sel**<br>1,95 ppm,m,2H; 2,2-2,8 ppm, m,4H; 3,0-3,7 ppm,m,6H; 4,3 ppm,m,2H; 7,0-7,3 ppm,m,10H; 7,7 ppm,td,1H; 7,95 ppm,m, 1H; 2H échangeables à 10,3 et 11,65 ppm |

TABLEAU I
SUITE VIII

| EXEMPLE N° | R | m | A | R1 | R2 | SPECTRE R.M.N. (SOLVANT) |
|---|---|---|---|---|---|---|
| 26 | 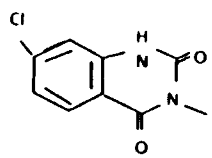 | 2 | 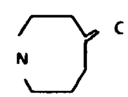 | 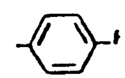 | 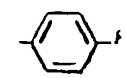 | RMN-$^1H$ ($DMSO-d_6$) sel 1,9 ppm,m,2H; 2,2-2,8 ppm, m,4H; 3,0-3,7 ppm,m,6H; 4,2 ppm,m,2H; 7,05-7,30 ppm,m, 10H; 7,9 ppm,d,1H; 2H échangeables à 10,1 et 11,8 ppm |
| 27 | 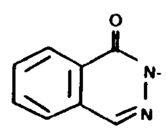 | 2 | 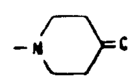 | 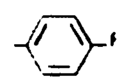 | 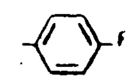 | RMN-$^1H$ ($CDCl_3$) base 2,6 ppm,m,2H; 2,85 ppm,m,2H; 3,05 ppm,m,2H; 3,5 pm,t,2H; 3,7 ppm,t,2H; 4,8 ppm,t,2H; 7,0 ppm,m,8H; 7,6-7,9 ppm,m,3H; 8,2 ppm,s,1H; 8,4 ppm,dd,1H |

**ETUDE PHARMACOLOGIOUE**

**EXEMPLE 28**

**Antagonisme à l'histamine**

Des cobayes albinos mâles (350-400 g), soumis à une diète hydrique pendant 18 heures avant l'essai, sont anesthésiés au carbamate d'éthyle à la dose de 1,25 g/kg par voie intrapéritonéale. Un catheter est introduit dans une artère carotide pour mesurer la pression artérielle au moyen d'une cellule de pression P23ID reliée à un enregistreur Gould 2400 ®. Un autre catheter est introduit dans une veine jugulaire et sert à injecter les composés à tester. La trachée est canulée et le cobaye est mis en respiration assistée à l'aide d'un respirateur Havard pour petits animaux.

La température du cobaye est maintenue aux environs de 37 °C à l'aide d'une lampe chauffante. Une aiguille piquée dans la canule trachéale est reliée à une cellule de pression P50 permettant d'enregistrer la pression trachéale.

Les cobayes sont prétraités par la d-Tubocurarine (1 mg/kg i.v.). On injecte ensuite l'histamine à la dose de 10 µg/kg par voie veineuse. Cette dose provoque une bronchoconstriction et entraîne une augmentation de la pression trachéale. Les injections de l'histamine sont répétées plusieurs fois à 10 minutes d'intervalle jusqu'à stabilisation de la réponse. Les composés de l'invention sont ensuite injectés par voie i.v. à doses cumulatives et la dose inhibant de 100% l'augmentation de la pression trachéale causée par l'injection de l'histamine ($ID_{100}$) est recherchée. L'$ID_{100}$ des composés de l'invention est entre 20 et 250 µg/kg.

**EXEMPLE 29**

**Antagonisme $5HT_2$**

Des rats Sprague-Dawley mâles (350-400 g) sont anesthésiés au pentobarbital par voie i.p. (45 mg/kg). La trachée est canulée et les animaux sont placés en respiration artificielle. Les nerfs vagues sont sectionnés. Un catheter est placé dans une artère carotide pour enregistrer la pression artérielle. Un second catheter est placé dans la veine du pénis et sert aux injections. On prend la température des animaux et on la maintient à 37 °C. Les rats sont prétraités par la d-tubocurarine (1 mg/kg i.v.) et la prazosine (1 mg/kg i.v.). La 5-hydroxytryptamine est injectée à la dose de 100 µg/kg i.v. 2 fois à 10 minutes d'intervalle de façon à déterminer l'élévation de la pression artérielle systolique de chaque rat. 10 minutes après, on injecte les composés de l'invention à la dose la plus faible et l'injection de la 5-hydroxytryptamine est refaite 10 minutes après. 3 ou 4 doses cumulatives des composés de l'invention sont testées de la même manière. On calcule les pourcentages de la réponse hypertensive, obtenus aux différentes doses afin de déterminer l'$ID_{50}$, dose inhibant de 50% la réponse hypertensive. Les résultats de cette étude sont indiqués dans le Tableau II.

TABLEAU II

| COMPOSE | $ID_{50}$ (µg/kg i.v.) |
|---|---|
| EXEMPLE 1 | 13,0 |
| EXEMPLE 3 | 24,0 |
| EXEMPLE 5 | 34,0 |
| EXEMPLE 6 | 39,0 |
| EXEMPLE 10 | 8,2 |
| EXEMPLE 13 | 27,0 |

**EXEMPLE 30**

**Antagonisme $\alpha_1$**

Des rats Sprague-Dawley de sexe mâle (300-400 g) ayant subi une diète hydrique, sont anesthésiés à l'éther éthylique. Une canule est placée dans la trachée. La moelle épinière est détruite au moyen d'une tige en acier et la respiration artificielle immédiatement commencée. Les nerfs vagues sont sectionnés. Les artères carotides sont ligaturées, un cathéter est placé dans l'une d'elles pour enregistrer la pression artérielle. Un second cathether est placé dans la veine du pénis et sert aux injections. On prend la température des animaux et on la maintient à 36 °C. Les rats sont prétraités par un $\beta$-bloquant (Tertatolol 100 $\mu$g/kg i.v.). La phényléphrine est injectée à la dose de 4 $\mu$g/kg i.v.. Deux injections identiques sont faites à 10 minutes d'intervalle. Les composés de l'invention sont injectés à la dose la plus faible et l'injection de phényléphrine est refaite 10 minutes après. 3 ou 4 doses cumulatives des composés de l'invention sont testées de la même manière. On calcule les pourcentages d'inhibition de la réponse hypertensive, obtenus aux différentes doses afin de déterminer l'$ID_{50}$.

Les résultats de cette étude sont donnés dans le Tableau III.

TABLEAU III

| COMPOSE | $ID_{50}$ ($\mu$g/kg) |
|---|---|
| EXEMPLE 1 | >5000 |
| EXEMPLE 3 | >5000 |
| EXEMPLE 4 | >5000 |
| EXEMPLE 8 | 5300 |
| EXEMPLE 9 | >5000 |
| EXEMPLE 11 | >5000 |
| EXEMPLE 13 | 3050 |
| EXEMPLE 23 | >5000 |
| EXEMPLE 24 | >5000 |
| EXEMPLE 25 | 3050 |

**EXEMPLE 31**

**Antagonisme 5HTP**

On utilise 4 rats Wistar femelles (270 ± 30 g) étant à jeun depuis environ 24 heures. Au début de l'essai on leur administre la solution "contrôle" ou les solutions des composés à tester et par gavage, on leur administre une solution de la 5-hydroxytryptophane à la dose de 320 mg/kg. Les animaux sont ensuite mis en observation dans des cages transparentes. Lors de l'étude trois paramètres sont évalués : Le "forepaw treading", le "flat body posture" et le "head-twitches". Le "forepaw treading" correspond à un pédalage des membres antérieurs. Ce paramètre est mesuré 80 minutes après l'administration de la 1,5-hydroxytrypthophane et pendant une période d'observation de dix minutes. Ce temps est divisé en périodes de 5 secondes et si un mouvement est observé sous cette période le score de 1 est noté, le maximum de score étant de 30 pour les 10 minutes d'observation et pour chaque animal.

Le paramètre "Head-twiches" est relatif au nombre de secousses de la tête des animaux observées pendant 10 minutes. Ce paramètre est évalué 90 minutes après l'administration de la 1,5-hydroxytryptophane et pendant une période de 10 minutes. Le "flat body posture" correspond à un aplatissement du corps qui dure plus de 10 minutes. Ce paramètre est évalué pendant tout le temps d'observation des animaux. Les résultats de ces études qui sont indiqués dans les Tableaux IV à VI démontrent que les composés de l'invention sont des antagonistes puissants de 5-HTP. Les résultats présentés dans le Tableau VI démontrent aussi que les composés de l'invention sont bien absorbés par voie orale, ce qui constitue un avantage

très important en thérapeutique.

TABLEAU IV

| Antagonisme 5-HTP Composés testés par voie intrapéritonéale | | | |
|---|---|---|---|
| COMPOSE | $DE_{50}$ (mg/kg) | | |
| | FPT * | FBP ** | HT *** |
| EXEMPLE 1 | 2.5 | 0.08 | 0.63 |
| EXEMPLE 2 | 1.25 | 1.25 | 5 |
| FPT * = fore-paw treading<br>FBP ** = flat body posture<br>HT *** = head-twitches | | | |

TABLEAU V

| Antagonisme 5-HTP Composés testés par voie subcutanée | | | |
|---|---|---|---|
| COMPOSE | $DE_{50}$ (mg/kg) | | |
| | FPT * | FBP ** | HT *** |
| EXEMPLE 3 | 10 | 10 | 10 |
| EXEMPLE 7 | 5 | 5 | >10 |
| EXEMPLE 10 | 1.25 | 0.16 | 40 |
| FPT * = fore-paw treading<br>FBP ** = flat body posture<br>HT *** = head-twitches | | | |

TABLEAU VI

| Antagonisme 5-HTP Composés testés par voie orale | | | |
|---|---|---|---|
| COMPOSE | $DE_{50}$ (mg/kg) | | |
| | FPT * | FBP ** | HT *** |
| EXEMPLE 1 | 10 | 0.16 | >10 |
| EXEMPLE 4 | 1.25 | 1.25 | 10 |
| EXEMPLE 6 | 5 | 2.5 | 5 |
| EXEMPLE 11 | 5 | 1.25 | 10 |
| FPT * = fore-paw treading<br>FBP ** = flat body posture<br>HT *** = head-twitches | | | |

**EXEMPLE 32**

**Comprimés dosés à 10 mg de chlorhydrate de la {[[bis(fluoro-4 phényl)méthylène]-4 pipéridino]-2 éthyl}-3 carbamoyl-8 hydroxy-4 méthyl-2 imidazo [1,5-a] pyrimidine [B.F.P.M.P.E.I.P.]**

| | |
|---|---|
| **B.F.P.M.P.E.I.P.** | **10 g** |
| **Amidon de blé** | **100 g** |
| **Amidon de mäis** | **20 g** |
| **Stéarate de magnésium** | **15 g** |
| **Talc** | **20 g** |
| **pour 1000 comprimés à 10 mg de principe actif.** | |

**Revendications**

**Revendications pour les Etats contractants suivants : AT, BE, CH, DE, DK, FR, GB, IT, LI, LU, NL, SE**

**1.** Composés de formule générale I :

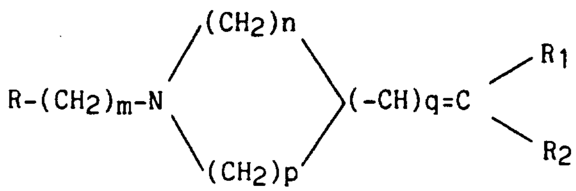

**(I)**

dans laquelle

- m représente un nombre entier de 2 à 4,
- n et p identiques ou différents représentent un nombre entier égal à 1, 2 ou 3, à la condition que la somme de n et de p soit supérieure ou égale à 3, et inférieure ou égale à 5,
- q représente 0 ou 1
- R représente un radical tétrahydro-1,2,3,4 quinazolyle-3 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-1,3 hexahydro-1,3,4,6,11,11a-2H pyrazino [1,2-b] isoquinolyle-2, un radical dihydro-1,2 oxo-1 phtalazinyle-2 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-2,6 pipérazinyle-1 de formule W :

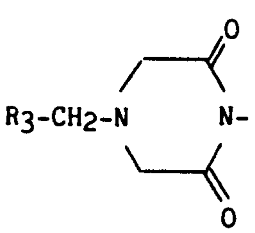

**(W)**

(dans laquelle $R_3$ représente un radical pyridyle-2 ou un radical phényle éventuellement substitué avec un ou plusieurs atomes d'halogène ou des radicaux alkyle ou alcoxy de 1 à 6 atomes de carbone),

un radical de formule Z :

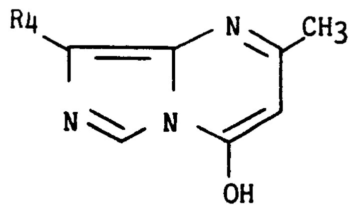

(Z)

(dans laquelle $R_4$ représente un radical carbamoyle, un radical cyano, un radical hydroxycarbonyle, ou un radical alcoxycarbonyle de 1 à 6 atomes de carbone),
ou un radical de formule Y :

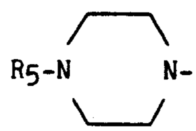

(Y)

(dans laquelle $R_5$ représente un radical pyrimidinyl-2, un radical isoquinolyl-1, un radical quinolyl-2, un radical pyridyl-2, un radical benzyle -éventuellement substitué par un radical alkyle de 1 à 6 atomes de carbone comportant un ou plusieurs atomes d'halogène-, un radical thiazolyl-2 -éventuellement substitué par un ou plusieurs radicaux alkyle de 1 à 6 atomes de carbone ou par un radical phényle-, ou un radical benzothiazolyl-2),

- $R_1$ et $R_2$

soit

identiques ou différents représentent chacun un radical phényle substitué par un ou plusieurs atomes d'halogène ou par un ou plusieurs radicaux alkyle ou alcoxy contenant de 1 à 6 atomes de carbone,

soit

$R_1$ représente un radical phényle et $R_2$ un radical pyridyle-2 (chacun de ces deux radicaux pouvant être substitué par un ou plusieurs atomes d'halogène ou par un ou plusieurs radicaux alkyle ou alcoxy contenant de 1 à 6 atomes de carbone),

soit

$R_1$ et $R_2$ forment ensemble avec l'atome de carbone auquel ils sont attachés un radical fluorène,

leurs stéréoisomères possibles et leurs sels d'addition à un acide minéral ou organique pharmaceutiquement acceptable.

**2.** La {[[bis(fluoro-4 phényl) méthylène]-4 pipéridino]-2 éthyl}-3 carbamoyl-8 hydroxy-4 méthyl-2 imidazo [1,5-a] pyrimidine, composé répondant à la formule générale I selon la revendication 1 et ses sels d'addition à un acide minéral ou organique, pharmaceutiquement acceptable.

**3.** La {[[bis(fluoro-4 phényl) méthylène]-4 pipéridino]-3 propyl}-1 (pyrimidinyl-2)-4 pipérazine, composé répondant à la formule générale I selon la revendication 1 et ses sels d'addition à un acide minéral ou organique, pharmaceutiquement acceptable.

**4.** La (fluoro-2 benzyl)-4 {[[(fluoro-4 phényl) phényl méthylène]-4 pipéridino]-3 propyl}-1 pipérazine dione-2,6, composé répondant à la formule générale I selon la revendication 1 et ses sels d'addition à un acide minéral ou organique, pharmaceutiquement acceptable.

**5.** La {[[(fluoro-4 phényl) phényl méthylène]-4 pipéridino]-3 propyl}-1 (pyridyl-2 méthyl)-4 pipérazine dione-2,6, composé répondant à la formule générale I selon la revendication 1 et ses sels d'addition à un acide minéral ou organique, pharmaceutiquement acceptable.

**6.** Procédé de préparation des composés de la formule générale I, selon la revendication 1, caractérisé en ce que :

**soit**

l'on condense

ou bien

une amine de formule générale II :

**RH (II)**

dans laquelle R a la même signification que pour la formule I selon la revendication 1, avec un composé de formule générale III :

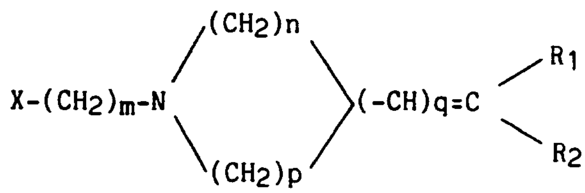

**(III)**

dans laquelle X représente un atome d'halogène, un radical mésyloxy, ou un radical tosyloxy et m, n, p, q, et $R_1$ et $R_2$, ont la même signification que, pour la formule I, selon la revendication 1, pour former les composés de formule générale I selon la revendication 1,

. ou bien

une amine de formule IV:

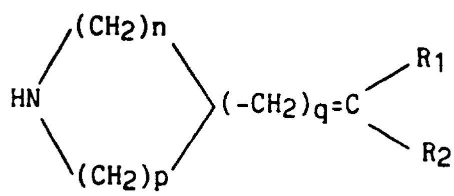

**(IV)**

dans laquelle $R_1$, $R_2$, n, p, q ont la même signification que pour la formule I, selon la revendication 1,

avec un composé de formule V :

**$R - (CH_2)_m - X$ (V)**

dans laquelle X a la signification indiquée pour la formule III et R et m ont la même signification que pour la formule I selon la revendication 1, pour former les composés de formule I,

**soit**

l'on cyclise un dérivé de l'amino-4 imidazole avec un composé de formule VI:

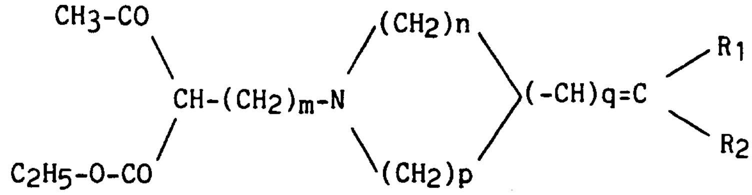

**(VI)**

dans laquelle m, n, p, q, $R_1$, $R_2$, ont la signification donnée pour la formule I, selon la revendication 1,

pour former les composés de la formule I dans laquelle R comprend un groupement imidazo [1,5a] pyrimidine et m, n, p, q, $R_1$, $R_2$ ont la signification indiquée pour la formule I, selon la revendication 1,

**lesquels ensuite**

si l'on désire, on sépare en leurs stéréoisomères possibles et/ou salifiés par un acide organique ou minéral, pharmaceutiquement acceptable pour former les sels d'addition correspondants.

**7.** Composition pharmaceutique contenant comme principe actif un composé selon l'une quelconque des revendications 1 à 5, en association ou en mélange avec un excipient ou un véhicule inerte non toxique, pharmaceutiquement acceptable.

**8.** Composition pharmaceutique selon la revendication 7 renfermant le principe actif à la dose de 0,5 à 100 mg.

**9.** Composition pharmaceutique selon les revendication 7 et 8 renfermant comme principe actif au moins un composé selon les revendications 1 à 5 utilisable dans le traitement des maladies nécessitant des antagonistes de l'histamine et de la sérotonine.

**10.** Les amines de formule $IV_b$ :

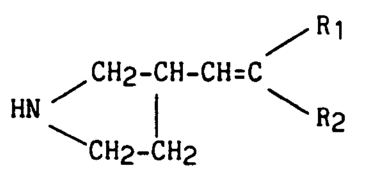

$(IV_b)$

dans laquelle $R_1$ et $R_2$ ont la signification indiquée pour la formule I selon la revendication 1, utiles comme intermédiaires à la préparation des composés de formule générale I selon la revendication 1.

**11.** Les amines de formule $IV_c$ :

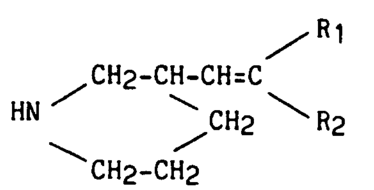

$(IV_c)$

dans laquelle $R_1$ et $R_2$ ont la signification indiquée pour la formule I selon la revendication 1, utiles comme intermédiaires à la préparation des composés de formule générale I selon la revendication 1.

**12.** Les amines de formule $IV_d$ :

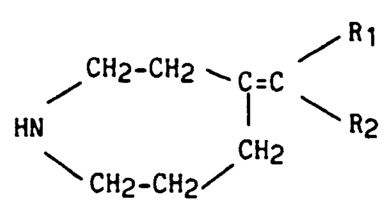

$(IV_d)$

dans laquelle $R_1$ et $R_2$ ont la signification indiquée pour la formule I selon la revendication 1, utiles

comme intermédiaires à la préparation des composés de formule générale I selon la revendication 1.

**Revendications pour l'Etat contractant suivant : ES**

**1.** Procédé de préparation des composés de formule générale I :

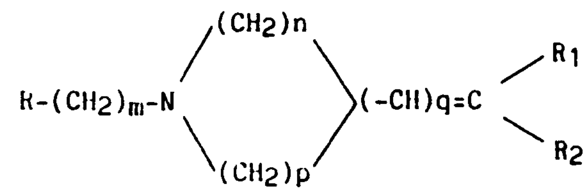

(I)

dans laquelle

- m représente un nombre entier de 2 à 4,
- n et p identiques ou différents représentent un nombre entier égal à 1, 2 ou 3, à la condition que la somme de n et de p soit supérieure ou égale à 3, et inférieure ou égale à 5,
- q représente 0 ou 1
- R représente un radical tetrahydro-1,2,3,4 quinazolyle-3 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-1,3 hexahydro-1,3,4,6,11,11a-2H pyrazino [1,2-b] isoquinolyle-2, un radical dihydro-1,2 oxo-1 phtalazinyle-2 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-2,6 pipérazinyle-1 de formule W :

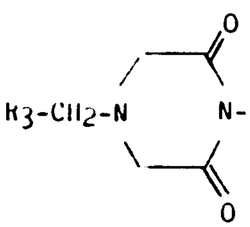

(W)

(dans laquelle $R_3$ représente un radical pyridyle-2 ou un radical phényle éventuellement substitué avec un ou plusieurs atomes d'halogène ou des radicaux alkyle ou alcoxy de 1 à 6 atomes de carbone),

un radical de formule Z :

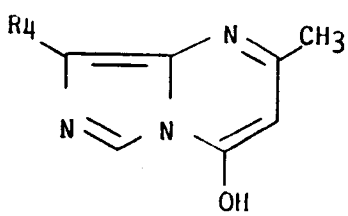

(Z)

(dans laquelle $R_4$ représente un radical carbamoyle, un radical cyano, un radical hydroxycarbonyle, ou un radical alcoxycarbonyle de 1 à 6 atomes de carbone),
ou un radical de formule Y :

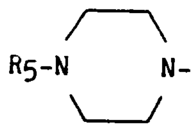

(Y)

(dans laquelle $R_5$ représente un radical pyrimidinyl-2, un radical isoquinolyl-1, un radical quinolyl-2, un radical pyridyl-2, un radical benzyle -éventuellement substitue par un radical alkyle de 1 à 6 atomes de carbone comportant un ou plusieurs atomes d'halogène-, un radical thiazolyl-2 -éventuellement substitué par un ou plusieurs radicaux alkyle de 1 à 6 atomes de carbone ou par un radical phényle-, ou un radical benzothiazolyl-2),

- $R_1$ et $R_2$

soit

identiques ou différents représentent chacun un radical phényle substitué par un ou plusieurs atomes d'halogène ou par un ou plusieurs radicaux alkyle ou alcoxy contenant de 1 à 6 atomes de carbone,

soit

$R_1$ représente un radical phényle et $R_2$ un radical pyridyle-2 (chacun de ces deux radicaux pouvant être substitué par un ou plusieurs atomes d'halogène ou par un ou plusieurs radicaux alkyle ou alcoxy contenant de 1 à 6 atomes de carbone),

soit

$R_1$ et $R_2$ forment ensemble avec l'atome de carbone auquel ils sont attachés un radical fluorène,

leurs stéréoisomères possibles et leurs sels d'addition à un acide minéral ou organique pharmaceutiquement acceptable,

caractérisé en ce que :

**soit**

l'on condense

ou bien

une amine de formule générale II :

**RH (II)**

dans laquelle R a la même signification que pour la formule I,

avec un composé de formule générale III :

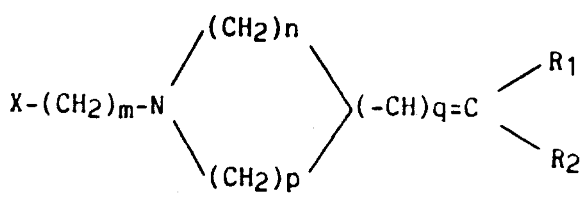

(III)

dans laquelle X représente un atome d'halogène, un radical mésyloxy, ou un radical tosyloxy et m, n, p, q, et $R_1$ et $R_2$, ont la même signification que, pour la formule I, pour former les composés de formule générale I,

. ou bien

une amine de formule IV:

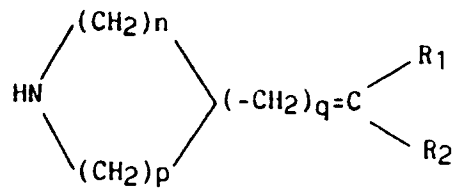

(IV)

dans laquelle $R_1$, $R_2$, n, p, q ont la même signification que pour la formule I, avec un compose de formule V :

**$R - (CH_2)_m - X$ (V)**

dans laquelle X a la signification indiquée pour la formule III et R et m ont la même signification que pour la formule I, pour former les composés de formule I,

**soit**

l'on cyclise un dérivé de l'amino-4 imidazole avec un compose de formule VI:

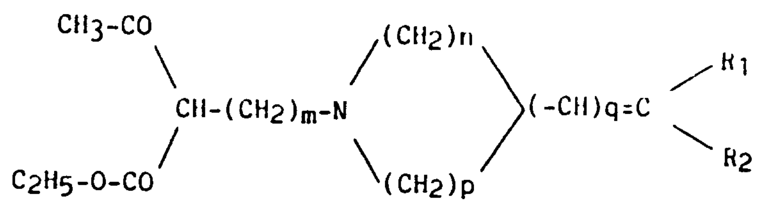

(VI)

dans laquelle m, n, p, q, $R_1$, $R_2$,ont la signification donnée pour la formule I,

pour former les composés de la formule I dans laquelle R comprend un groupement imidazo [1,5a] pyrimidine et m, n, p, q, $R_1$, $R_2$ ont la signification indiquée pour la formule I,

**lesquels ensuite**

si l'on désire, on sépare en leurs stéréoisomères possibles et/ou salifiés par un acide organique ou minéral, pharmaceutiquement acceptable pour former les sels d'addition correspondants.

2. La {[[bis(fluoro-4 phényl) méthylène]-4 pipéridino]-2 éthyl}-3 carbamoyl-8 hydroxy-4 méthyl-2 imidazo [1,5-a] pyrimidine, compose répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

3. La {[[bis(fluoro-4 phényl) méthylène]-4 pipéridino]-3 propyl}-1 (pyrimidinyl-2)-4 pipérazine, composé répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

4. La (fluoro-2 benzyl)-4 {[[(fluoro-4 phényl) phényl méthylène]-4 pipéridino]-3 propyl}-1 pipérazine dione-2,6, composé répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

5. La {[[(fluoro-4 phényl) phényl méthylène]-4 pipéridino]-3 propyl}-1 (pyridyl-2 méthyl)-4 pipérazine dione-2,6, composé répondant à la formule générale I selon la revendication 1 et ses sels d'addition à un acide minéral ou organique, pharmaceutiquement acceptable, lorsque préparée selon le procédé de

la revendication 1 ou un procédé chimique équivalent.

**6.** Les amines de formule $IV_b$ :

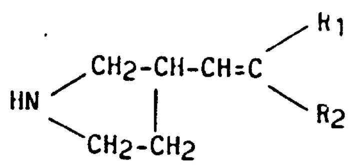

(IVb)

dans laquelle $R_1$ et $R_2$ ont la signification indiquée pour la formule I selon la revendication 1, utiles comme intermédiaires à la préparation des composés de formule générale I selon la revendication 1.

**7.** Les amines de formule $IV_c$ :

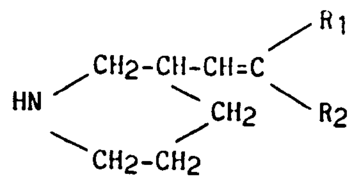

(IVc)

dans laquelle $R_1$ et $R_2$ ont la signification indiquée pour la formule I selon la revendication 1, utiles comme intermédiaires à la préparation des composés de formule générale I selon la revendication 1.

**8.** Les amines de formule $IV_d$ :

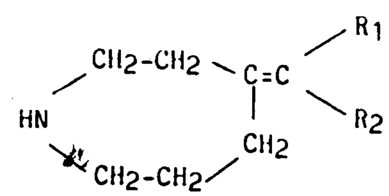

(IVd)

dans laquelle $R_1$ et $R_2$ ont la signification indiquée pour la formule I selon la revendication 1, utiles comme intermédiaires à la préparation des composés de formule générale I selon la revendication 1.

**Revendications pour l'Etat contractant suivant : GR**

**1.** Procédé de préparation des composés de formule générale I :

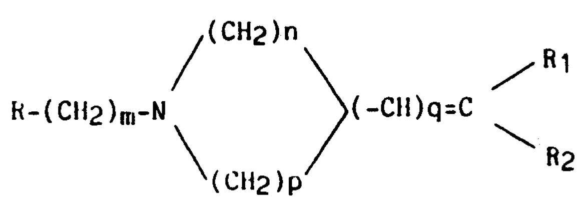

(I)

dans laquelle

- m représente un nombre entier de 2 à 4,
- n et p identiques ou différents représentent un nombre entier égal à 1, 2 ou 3, à la condition que la somme de n et de p soit supérieure ou égale à 3, et inférieure ou égale à 5,
- q représente 0 ou 1

- R représente un radical tetrahydro-1,2,3,4 quinazolyle-3 (éventuellement substitué sur le cycle benzénique par un ou plusieurs atomes d'halogènes, par des radicaux alkyle de 1 à 6 atomes de carbone un des radicaux alcoxy de 1 à 6 atomes de carbone), un radical dioxo-1,3 hexahydro-1,3,4,6,11,11a-2H pyrazino [1,2-b] isoquinolyle-2, un radical dihydro-1,2 oxo-1 phtalazinyle-2 (éventuellement substitué sur le cycle benzénique par un ou plusieurs atomes d'halogènes, par des radicaux alkyle de 1 à 6 atomes de carbone ou des radicaux alcoxy de 1 à 6 atomes de carbone), un radical dioxo-2,6 pipérazinyle-1 de formule W :

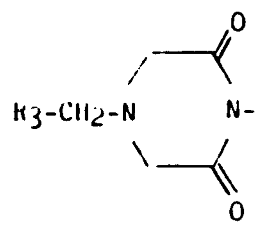

(W)

(dans laquelle $R_3$ représente un radical pyridyle-2 ou un radical phényle éventuellement substitué avec un ou plusieurs atomes d'halogène ou des radicaux alkyle au alcoxy de 1 à 6 atomes de carbone),

un radical de formule Z :

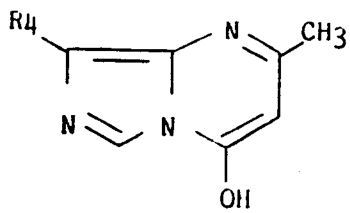

(Z)

(dans laquelle $R_4$ représente un radical carbamoyle, un radical cyano, un radical hydroxycarbonyle, ou un radical alcoxycarbonyle de 1 à 6 atomes de carbone),

ou un radical de formule Y :

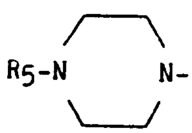

(Y)

(dans laquelle $R_5$ représente un radical pyrimidinyl-2, un radical isoquinolyl-1, un radical quinolyl-2, un radical pyridyl-2, un radical benzyle -éventuellement substitué par un radical alkyle de 1 à 6 atomes de carbone comportant un ou plusieurs atomes d'halogène-, un radical thiazolyl-2 -éventuellement substitué par un ou plusieurs radicaux alkyle de 1 à 6 atomes de carbone ou par un radical phényle-, ou un radical benzothiazolyl-2),

- $R_1$ et $R_2$

soit

identiques ou différents représentent chacun un radical phényle substitué par un ou plusieurs atomes d'halogène ou par un ou plusieurs radicaux alkyle ou alcoxy contenant de 1 à 6 atomes de carbone,

soit

$R_1$ représente un radical phényle et $R_2$ un radical pyridyle-2 (chacun de ces deux radicaux pouvant être substitué par un ou plusieurs atomes d'halogène ou par un ou plusieurs radicaux alkyle ou alcoxy contenant de 1 à 6 atomes de carbone),

soit

$R_1$ et $R_2$ forment ensemble avec l'atome de carbone auquel ils sont attachés un radical fluorène,

leurs stéréoisomères possibles et leurs sels d'addition à un acide minéral ou organique pharmaceutiquement acceptable,
caractérisé en ce que :

**soit**

l'on condense
ou bien
une amine de formule générale II :

**RH (II)**

dans laquelle R a la même signification que pour la formule I,
avec un composé de formule générale III :

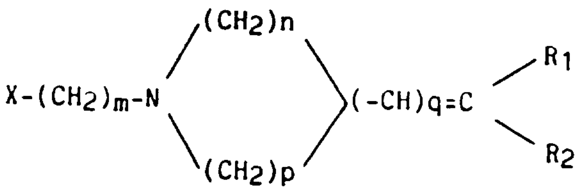

(III)

dans laquelle X représente un atome d'halogène, un radical mésyloxy, ou un radical tosyloxy et m, n, p, q, et $R_1$ et $R_2$, ont la même signification que, pour la formule I, pour former les composés de formule générale I,
. ou bien
une amine de formule IV:

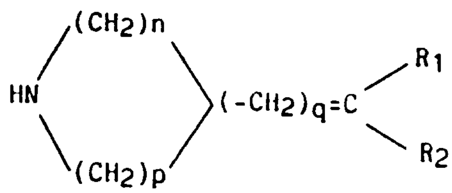

(IV)

dans laquelle $R_1$, $R_2$, n, p, q, ont la même signification que pour la formule I,
avec un compose de formule V :

**R - $(CH_2)$m - X (V)**

dans laquelle X a la signification indiquée pour la formule III et R et m ont la même signification que pour la formule I, pour former les composés de formule I,

**soit**

l'on cyclise un dérivé de l'amino-4 imidazole avec un composé de formule VI:

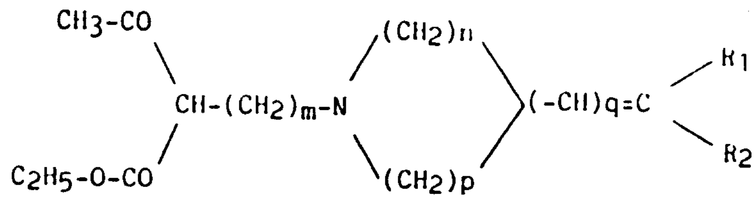

(VI)

dans laquelle m, n, p, q, $R_1$, $R_2$,ont la signification donnée pour la formule I,

pour former les composés de la formule I dans laquelle R comprend un groupement imidazo [1,5a] pyrimidine et m, n, p, q, $R_1$, $R_2$ ont la signification indiquée pour la formule I,

**lesquels ensuite**

si l'on désire, on sépare en leurs stéréoisomères possibles et/ou salifiés par un acide organique ou mineral, pharmaceutiquement acceptable pour former les sels d'addition correspondants.

2. La {[[bis(fluoro-4 phényl) méthylène]-4 pipéridino]-2 éthyl}-3 carbamoyl-8 hydroxy-4 méthyl-2 imidazo [1,5-a] pyrimidine, compose répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

3. La {[[bis(fluoro-4 phényl) méthylène]-4 pipéridino]-3 propyl}-1 (pyrimidinyl-2)-4 pipérazine, composé répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

4. La (fluoro-2 benzyl)-4 {[[(fluoro-4 phényl) phényl méthylène]-4 pipéridino]-3 propyl}-1 pipérazine dione-2,6, composé répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

5. La {[[(fluoro-4 phényl) phényl méthylène]-4 pipéridino]-3 propyl}-1 (pyridyl-2 méthyl)-4 pipérazine dione-2,6, composé répondant à la formule générale I selon la revendication 1 et ses sels d'addition à un acide minéral ou organique, pharmaceutiquement acceptable, lorsque préparée selon le procédé de la revendication 1 ou un procédé chimique équivalent.

6. Les amines de formule $IV_b$ :

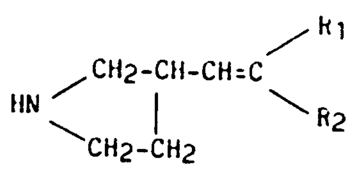

($IV_b$)

dans laquelle $R_1$ et $R_2$ ont la signification indiquée pour la formule I selon la revendication 1, utiles comme intermédiaires à la préparation des composés de formule générale I selon la revendication 1.

**7.** Les amines de formule $IV_c$ :

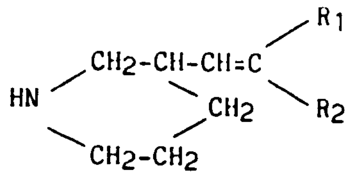

($IV_c$)

dans laquelle $R_1$ et $R_2$ ont la signification indiquée pour la formule I selon la revendication 1, utiles comme intermédiaires à la préparation des composés de formule générale I selon la revendication 1.

**8.** Les amines de formule $IV_d$ :

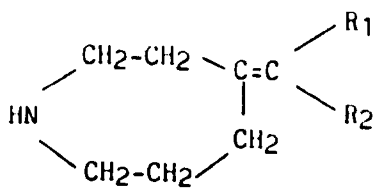

($IV_d$)

dans laquelle $R_1$ et $R_2$ ont la signification indiquée pour la formule I selon la revendication 1, utiles comme intermédiaires à la préparation des composés de formule générale I selon la revendication 1.

**Claims**
**Claims for the following Contracting States : AT, BE, CH, DE, DK, FR, GB, IT, LI, LU, NL, SE**

**1.** Compounds of the general formula I :

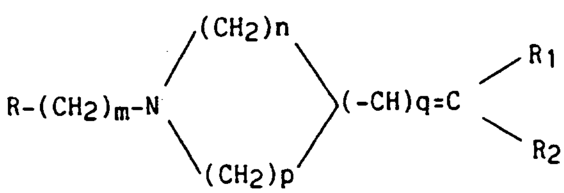

(I)

in which
- m represents an integer of from 2 to 4,
- n and p are the same or different and represent an integer 1, 2 or 3, with the proviso that the sum of n and p is greater than or equal to 3, and less than or equal to 5,
- q represents 0 or 1,
- R represents a 1,2,3,4-tetrahydroquinazol-3-yl radical (optionally substituted at the benzene ring by one or more halogen atoms, by alkyl radicals containing from 1 to 6 carbon atoms or alkoxy radicals containing from 1 to 6 carbon atoms), a 1,3-dioxo-1,3,4,6,11,11a-hexahydro-2H-pyrazino-[1,2-b]isoquinol-2-yl radical, a 1,2-dihydro-1-oxo-phthalazin-2-yl radical (optionally substituted at the benzene ring by one or more halogen atoms, by alkyl radicals containing from 1 to 6 carbon atoms or alkoxy radicals containing from 1 to 6 carbon atoms), a 2,6-dioxopiperazin-1-yl radical of formula W :

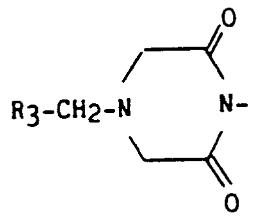

(W)

(in which $R_3$ represents a pyrid-2-yl radical or a phenyl radical optionally substituted by one or more halogen atoms or alkyl or alkoxy radicals containing from 1 to 6 carbon atoms),
a radical of formula Z :

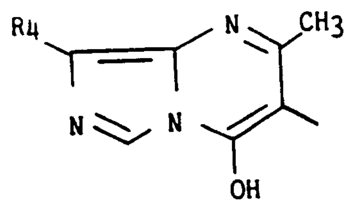

(Z)

(in which $R_4$ represents a carbamoyl radical, a cyano radical, a hydroxycarbonyl radical, or an alkoxycarbonyl radical containing from 1 to 6 carbon atoms),
or a radical of formula Y :

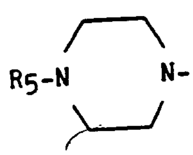

(Y)

(in which $R_5$ represents a pyrimidin-2-yl radical, an isoquinol-1-yl radical, a quinol-2-yl radical, a pyrid-2-yl radical, a benzyl radical - optionally substituted by an alkyl radical having from 1 to 6 carbon atoms and containing one or more halogen atoms - , a thiazol-2-yl radical optionally substituted by one or more alkyl radicals containing from 1 to 6 carbon atoms or by a phenyl radical, or a benzothiazol-2-yl radical),

- $R_1$ and $R_2$

either

are the same or different and each represents a phenyl radical substituted by one or more halogen atoms or by one or more alkyl or alkoxy radicals containing from 1 to 6 carbon atoms,

or

$R_1$ represents a phenyl radical and $R_2$ represents a pyrid-2-yl radical (it being possible for each of those radicals to be substituted by one or more halogen atoms or by one or more alkyl or alkoxy radicals containing from 1 to 6 carbon atoms),

or

$R_1$ and $R_2$, together with the carbon atom to which they are attached, form a fluorene radical,

their possible stereoisomers and their addition salts with a pharmaceutically acceptable mineral or organic acid.

**2.** 3-{2-[4-[bis(4-fluorophenyl)methylene]piperidino]ethyl}-8-carbamoyl-4-hydroxy-2-methylimidazo[1,5-a]-pyrimidine, a compound corresponding to the general formula I according to claim 1, and its addition salts with a pharmaceutically acceptable mineral or organic acid.

**3.** 1-{3-[4-[bis(4-fluorophenyl)methylene]piperidino]propyl}-4-(pyrimidin-2-yl)piperazine, a compound corresponding to the general formula I according to claim 1, and its addition salts with a pharmaceutically acceptable mineral or organic acid.

**4.** 4-(2-fluorobenzyl)-1-{3-[4-[(4-fluorophenyl)phenylmethylene]piperidino]propyl}piperazine-2,6-dione, a compound corresponding to the general formula I according to claim 1, and its addition salts with a pharmaceutically acceptable mineral or organic acid.

**5.** 1-{3-[4-[(4-fluorophenyl)phenylmethylene]piperidino]propyl}-4-(pyrid-2-ylmethyl)piperazine-2,6-dione, a compound corresponding to the general formula I according to claim 1, and its addition salts with a pharmaceutically acceptable mineral or organic acid.

**6.** Process for the preparation of compounds of the general formula I according to claim 1, characterised in that :

**either**

an amine of the general formula II :

RH (II)

in which R is as defined for formula I according to claim 1, is condensed with a compound of the general formula III :

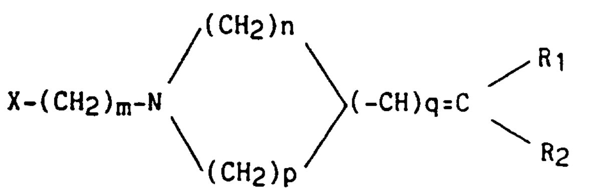

(III)

in which X represents a halogen atom, a mesyloxy radical or a tosyloxy radical, and m, n, p, q and $R_1$ and $R_2$ are as defined for formula I according to claim 1, to form compounds of the general formula I according to claim 1,

• or

an amine of formula IV

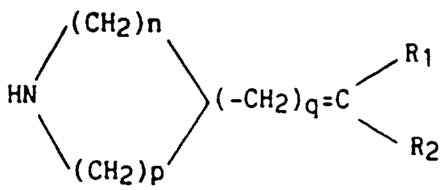

(IV)

in which $R_1$, $R_2$, n, p and q are as defined for formula I according to claim 1, is condensed with a compound of formula V :

R - $(CH_2)_m$ - X (V)

in which X is as defined for formula III and R and m are as defined for formula I according to claim 1, to form compounds of formula I,

**or**

a 4-aminoimidazole compound is cyclised with a compound of formula VI:

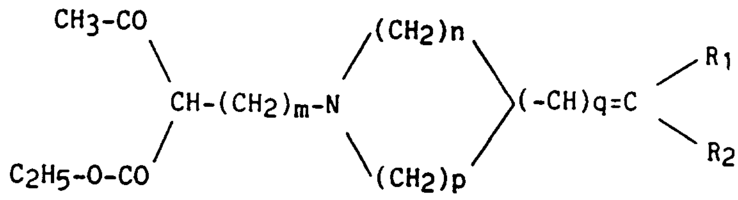

(VI)

in which m, n, p, q, $R_1$ and $R_2$ are as defined for formula I according to claim 1, to form compounds of formula I in which R contains an imidazo[1,5a]pyrimidine group and m, n, p, q, $R_1$ and $R_2$ are as defined for formula I according to claim 1,

which are then

if desired, separated into their possible stereoisomers and/or converted into salts with a pharmaceutically acceptable organic or mineral acid to form the corresponding addition salts.

**7.** Pharmaceutical composition comprising as active ingredient a compound according to any one of claims 1 to 5, in association with or in a mixture with a pharmaceutically acceptable, inert, non-toxic excipient or carrier.

**8.** Pharmaceutical composition according to claim 7, containing the active ingredient in an amount of from 0.5 to 100 mg.

**9.** Pharmaceutical composition according to claims 7 and 8 containing as active ingredient at least one compound according to any one of claims 1 to 5 which can be used in the treatment of disorders requiring antagonists of histamine and serotonin.

**10.** The amines of formula $IV_b$ :

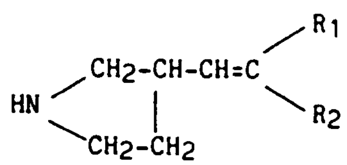

(IVb)

in which $R_1$ and $R_2$ are as defined for formula I according to claim 1, which are useful as intermediates in the preparation of compounds of the general formula I according to claim 1.

**11.** The amines of formula $IV_c$ :

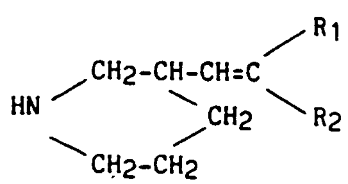

(IVc)

in which $R_1$ and $R_2$ are as defined for formula I according to claim 1, which are useful as intermediates in the preparation of compounds of the general formula I according to claim 1.

**12.** The amines of formula $IV_d$ :

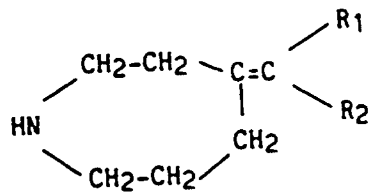

(IVd)

in which $R_1$ and $R_2$ are as defined for formula I according to claim 1, which are useful as intermediates in the preparation of compounds of the general formula I according to claim 1.

**Claims for the following Contracting State : ES**

**1.** Process for the preparation of compounds of the general formula I :

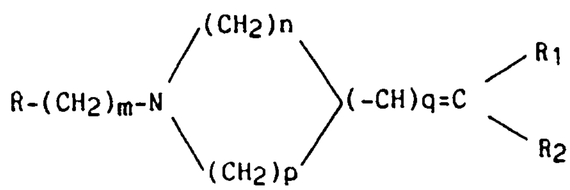

(I)

in which

- m represents an integer of from 2 to 4,
- n and p are the same or different and represent an integer 1, 2 or 3, with the proviso that the sum of n and p is greater than or equal to 3, and less than or equal to 5,
- q represents 0 or 1,
- R represents a 1,2,3,4-tetrahydroquinazol-3-yl radical (optionally substituted at the benzene ring by one or more halogen atoms, by alkyl radicals containing from 1 to 6 carbon atoms or alkoxy radicals containing from 1 to 6 carbon atoms), a 1,3-dioxo-1,3,4,6,11,11a-hexahydro-2H-pyrazino-[1,2-b]isoquinol-2-yl radical, a 1,2-dihydro-1-oxo-phthalazin-2-yl radical (optionally substituted at the benzene ring by one or more halogen atoms, by alkyl radicals containing from 1 to 6 carbon atoms or alkoxy radicals containing from 1 to 6 carbon atoms), a 2,6-dioxopiperazin-1-yl radical of formula W :

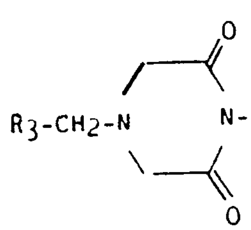

(W)

(in which $R_3$ represents a pyrid-2-yl radical or a phenyl radical optionally substituted by one or more halogen atoms or alkyl or alkoxy radicals containing from 1 to 6 carbon atoms),

a radical of formula Z :

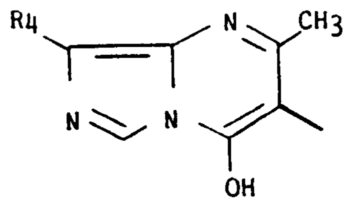

(Z)

(in which $R_4$ represents a carbamoyl radical, a cyano radical, a hydroxycarbonyl radical, or an alkoxycarbonyl radical containing from 1 to 6 carbon atoms),
or a radical of formula Y :

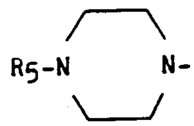

(Y)

(in which $R_5$ represents a pyrimidin-2-yl radical, an isoquinol-1-yl radical, a quinol-2-yl radical, a pyrid-2-yl radical, a benzyl radical - optionally substituted by an alkyl radical having from 1 to 6 carbon atoms and containing one or more halogen atoms - , a thiazol-2-yl radical optionally substituted by one or more alkyl radicals containing from 1 to 6 carbon atoms or by a phenyl radical, or a benzothiazol-2-yl radical),

- $R_1$ and $R_2$

either

are the same or different and each represents a phenyl radical substituted by one or more halogen atoms or by one or more alkyl or alkoxy radicals containing from 1 to 6 carbon atoms,

or

$R_1$ represents a phenyl radical and $R_2$ represents a pyrid-2-yl radical (it being possible for each of those radicals to be substituted by one or more halogen atoms or by one or more alkyl or alkoxy radicals containing from 1 to 6 carbon atoms),

or

$R_1$ and $R_2$, together with the carbon atom to which they are attached, form a fluorene radical,

their possible stereoisomers and their addition salts with a pharmaceutically acceptable mineral or organic acid,

characterised in that :

**either**

an amine of the general formula II :

RH (II)

in which R is as defined for formula I, is condensed with a compound of the general formula III :

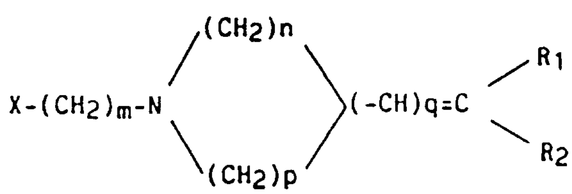

(III)

in which X represents a halogen atom, a mesyloxy radical or a tosyloxy radical, and m, n, p, q and

$R_1$ and $R_2$ are as defined for formula I, to form compounds of the general formula I,

• or

an amine of formula IV

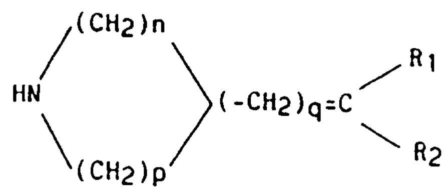

(IV)

in which $R_1$, $R_2$, n, p and q are as defined for formula I, is condensed with a compound of formula V :

$R - (CH_2)_m - X$ (V)

in which X is as defined for formula III and R and m are as defined for formula I, to form compounds of formula I,

or

a 4-aminoimidazole compound is cyclised with a compound of formula VI:

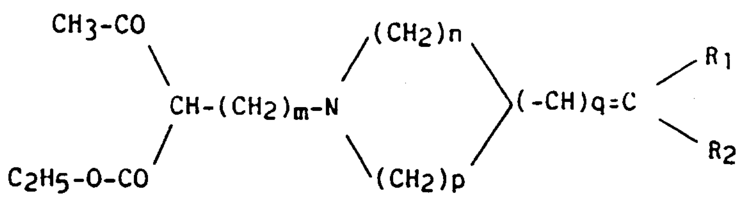

(VI)

in which m, n, p, q, $R_1$ and $R_2$ are as defined for formula I,

to form compounds of formula I in which R contains an imidazo[1,5a]pyrimidine group and m, n, p, q, $R_1$ and $R_2$ are as defined for formula I,

which are then

if desired, separated into their possible stereoisomers and/or converted into salts with a pharmaceutically acceptable organic or mineral acid to form the corresponding addition salts.

**2.** 3-{2-[4-[bis(4-fluorophenyl)methylene]piperidino]ethyl}-8-carbamoyl-4-hydroxy-2-methylimidazo[1,5-a]-pyrimidine, a compound corresponding to the general formula I according to claim 1, and its addition salts with a pharmaceutically acceptable mineral or organic acid, when prepared in accordance with the process of claim 1 or an equivalent chemical process.

**3.** 1-{3-[4-[bis(4-fluorophenyl)methylene]piperidino]propyl}-4-(pyrimidin-2-yl)piperazine, a compound corresponding to the general formula I according to claim 1, and its addition salts with a pharmaceutically acceptable mineral or organic acid, when prepared in accordance with the process of claim 1 or an equivalent chemical process.

**4.** 4-(2-fluorobenzyl)-1-{3-[4-[(4-fluorophenyl)phenylmethylene]piperidino]propyl}piperazine-2,6-dione, a compound corresponding to the general formula I according to claim 1, and its addition salts with a pharmaceutically acceptable mineral or organic acid, when prepared in accordance with the process of

claim 1 or an equivalent chemical process.

5. 1-{3-[4-[(4-fluorophenyl)phenylmethylene]piperidino]propyl}-4-(pyrid-2-ylmethyl)piperazine-2,6-dione, a compound corresponding to the general formula I according to claim 1, and its addition salts with a pharmaceutically acceptable mineral or organic acid, when prepared in accordance with the process of claim 1 or an equivalent chemical process.

6. The amines of formula $IV_b$ :

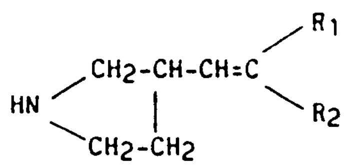

(IVb)

in which $R_1$ and $R_2$ are as defined for formula I according to claim 1, which are useful as intermediates in the preparation of compounds of the general formula I according to claim 1.

7. The amines of formula $IV_c$ :

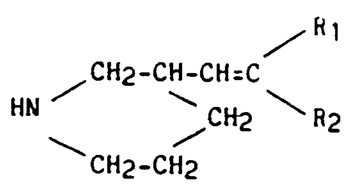

(IVc)

in which $R_1$ and $R_2$ are as defined for formula I according to claim 1, which are useful as intermediates in the preparation of compounds of the general formula I according to claim 1.

8. The amines of formula $IV_d$ :

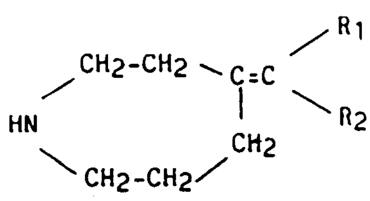

(IVd)

in which $R_1$ and $R_2$ are as defined for formula I according to claim 1, which are useful as intermediates in the preparation of compounds of the general formula I according to claim 1.

**Claims for the following Contracting State : GR**

1. Process for the preparation of compounds of the general formula I :

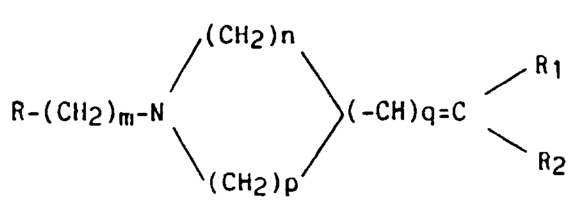

(I)

in which

- m represents an integer of from 2 to 4,
- n and p are the same or different and represent an integer 1, 2 or 3, with the proviso that the sum of n and p is greater than or equal to 3, and less than or equal to 5,
- q represents 0 or 1,
- R represents a 1,2,3,4-tetrahydroquinazol-3-yl radical (optionally substituted at the benzene ring by one or more halogen atoms, by alkyl radicals containing from 1 to 6 carbon atoms or alkoxy radicals containing from 1 to 6 carbon atoms), a 1,3-dioxo-1,3,4,6,11,11a-hexahydro-2H-pyrazino-[1,2-b]isoquinol-2-yl radical, a 1,2-dihydro-1-oxo-phthalazin-2-yl radical (optionally substituted at the benzene ring by one or more halogen atoms, by alkyl radicals containing from 1 to 6 carbon atoms or alkoxy radicals containing from 1 to 6 carbon atoms), a 2,6-dioxopiperazin-1-yl radical of formula W :

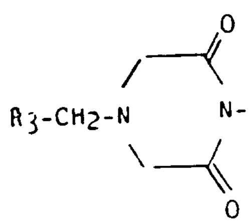

(W)

(in which $R_3$ represents a pyrid-2-yl radical or a phenyl radical optionally substituted by one or more halogen atoms or alkyl or alkoxy radicals containing from 1 to 6 carbon atoms),
a radical of formula Z :

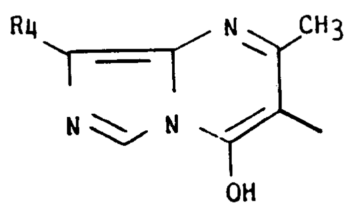

(Z)

(in which $R_4$ represents a carbamoyl radical, a cyano radical, a hydroxycarbonyl radical, or an alkoxycarbonyl radical containing from 1 to 6 carbon atoms),
or a radical of formula Y :

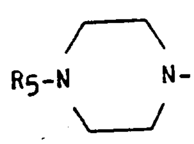

(Y)

(in which $R_5$ represents a pyrimidin-2-yl radical, an isoquinol-1-yl radical, a quinol-2-yl radical, a pyrid-2-yl radical, a benzyl radical - optionally substituted by an alkyl radical having from 1 to 6 carbon atoms and containing one or more halogen atoms - , a thiazol-2-yl radical optionally substituted by one or more alkyl radicals containing from 1 to 6 carbon atoms or by a phenyl radical, or a benzothiazol-2-yl radical),

- $R_1$ and $R_2$

either

are the same or different and each represents a phenyl radical substituted by one or more halogen atoms or by one or more alkyl or alkoxy radicals containing from 1 to 6 carbon atoms,

or

$R_1$ represents a phenyl radical and $R_2$ represents a pyrid-2-yl radical (it being possible for each of those radicals to be substituted by one or more halogen atoms or by one or more alkyl or alkoxy radicals containing from 1 to 6 carbon atoms),

or

$R_1$ and $R_2$, together with the carbon atom to which they are attached, form a fluorene radical,

their possible stereoisomers and their addition salts with a pharmaceutically acceptable mineral or organic acid,

characterised in that :

**either**

an amine of the general formula II :

RH (II)

in which R is as defined for formula I, is condensed with a compound of the general formula III :

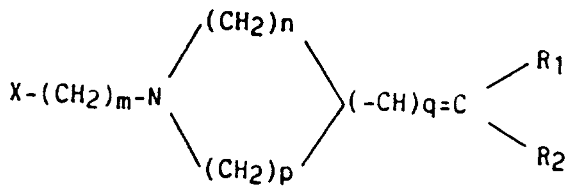

(III)

in which X represents a halogen atom, a mesyloxy radical or a tosyloxy radical, and m, n, p, q and $R_1$ and $R_2$ are as defined for formula I, to form compounds of the general formula I,

• or

an amine of formula IV

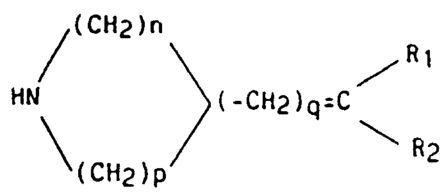

(IV)

in which $R_1$, $R_2$, n, p and q are as defined for formula I, is condensed with a compound of formula V :

$R - (CH_2)_m - X$ (V)

in which X is as defined for formula III and R and m are as defined for formula I, to form compounds of formula I,

**or**

a 4-aminoimidazole compound is cyclised with a compound of formula VI:

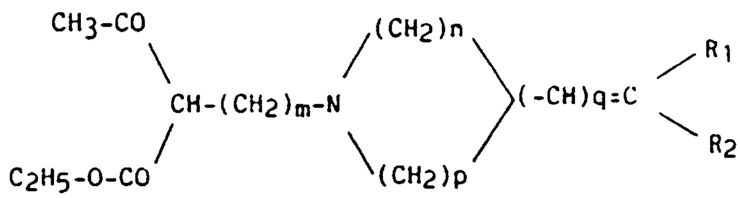

(VI)

in which m, n, p, q, $R_1$ and $R_2$ are as defined for formula I,

to form compounds of formula I in which R contains an imidazo[1,5a]pyrimidine group and m, n, p, q, $R_1$ and $R_2$ are as defined for formula I,

which are then

if desired, separated into their possible stereoisomers and/or converted into salts with a pharmaceutically acceptable organic or mineral acid to form the corresponding addition salts.

**2.** 3-{2-[4-[bis(4-fluorophenyl)methylene]piperidino]ethyl}-8-carbamoyl-4-hydroxy-2-methylimidazo[1,5-a]-pyrimidine, a compound corresponding to the general formula I according to claim 1, and its addition salts with a pharmaceutically acceptable mineral or organic acid, when prepared in accordance with the process of claim 1 or an equivalent chemical process.

**3.** 1-{3-[4-[bis(4-fluorophenyl)methylene]piperidino]propyl}-4-(pyrimidin-2-yl)piperazine, a compound corresponding to the general formula I according to claim 1, and its addition salts with a pharmaceutically acceptable mineral or organic acid, when prepared in accordance with the process of claim 1 or an equivalent chemical process.

**4.** 4-(2-fluorobenzyl)-1-{3-[4-[(4-fluorophenyl)phenylmethylene]piperidino]propyl}piperazine-2,6-dione, a compound corresponding to the general formula I according to claim 1, and its addition salts with a pharmaceutically acceptable mineral or organic acid, when prepared in accordance with the process of claim 1 or an equivalent chemical process.

**5.** 1-{3-[4-[(4-fluorophenyl)phenylmethylene]piperidino]propyl}-4-(pyrid-2-ylmethyl)piperazine-2,6-dione, a compound corresponding to the general formula I according to claim 1, and its addition salts with a pharmaceutically acceptable mineral or organic acid, when prepared in accordance with the process of claim 1 or an equivalent chemical process.

**6.** The amines of formula $IV_b$ :

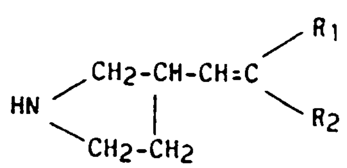

(IVb)

in which $R_1$ and $R_2$ are as defined for formula I according to claim 1, which are useful as intermediates in the preparation of compounds of the general formula I according to claim 1.

**7.** The amines of formula $IV_c$ :

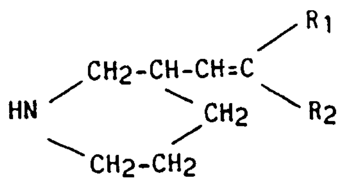

(IVc)

in which $R_1$ and $R_2$ are as defined for formula I according to claim 1, which are useful as intermediates in the preparation of compounds of the general formula I according to claim 1.

**8.** The amines of formula $IV_d$ :

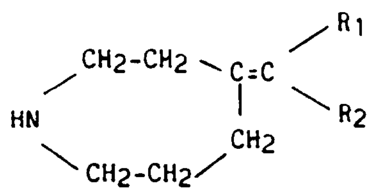

(IVd)

in which $R_1$ and $R_2$ are as defined for formula I according to claim 1, which are useful as intermediates in the preparation of compounds of the general formula I according to claim 1.

**Patentansprüche**

**Patentansprüche für folgende Vertragsstaaten : AT, BE, CH, DE, DK, FR, GB, IT, LI, LU, NL, SE**

**1.** Verbindungen der allgemeinen Formel I:

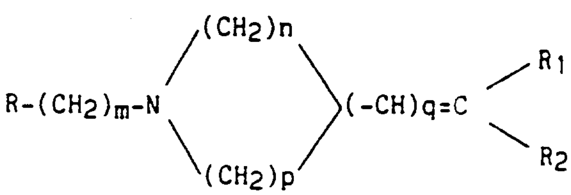

(I)

in der

- m eine ganze Zahl mit einem Wert von 2 bis 4,
- n und p, die gleichartig oder verschieden sein können, ganze Zahlen mit den Werten von 1, 2 oder 3 mit der Maßgabe, daß die Summe von n und p größer oder gleich 3 und kleiner oder gleich 5 ist,
- q 0 oder 1,
- R eine 1,2,3,4-Tetrahydro-chinazol-3-yl-gruppe (die gegebenenfalls am Benzolring durch ein oder mehrere Halogenatome, Alkylgruppen mit 1 bis 6 Kohlenstoffatomen oder Alkoxygruppen mit 1 bis 6 Kohlenstoffatomen substituiert ist), eine 1,3-Dioxo-1,3,4,6,11,11a-hexahydro-2H-pyrazino[1,2-b]isochinol-2-yl-gruppe, eine 1,2-Dihydro-1-oxo-phthalazin-2-yl-gruppe (die gegebenenfalls am Benzolring durch ein oder mehrere Halogenatme, Alkylgruppen mit 1 bis 6 Kohlenstoffatomen oder Alkoxygruppen mit 1 bis 6 Kohlenstoffatomen substituiert ist), eine 2,6-Dioxo-piperazin-1-yl-gruppe der Formel W:

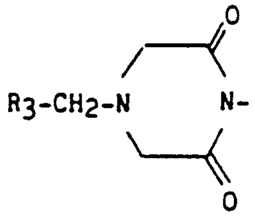

(W)

(in der $R_3$ eine Pyrid-2-yl-gruppe oder eine Phenylgruppe, die gegebenenfalls mit ein oder mehreren Halogenatomen oder Alkylgruppen oder Alkoxygruppen mit 1 bis 6 Kohlenstoffatomen substituiert ist, darstellt),
eine Gruppe der Formel Z:

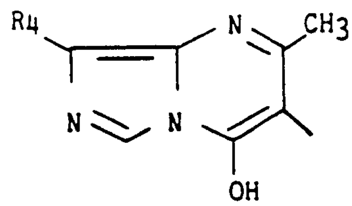

(Z)

(in der $R_4$ eine Carbamoylgruppe, eine Cyanogruppe, eine Hydroxycarbonylgruppe oder eine Alkoxycarbonylgruppe mit 1 bis 6 Kohlenstoffatomen darstellt),
oder eine Gruppe der Formel Y:

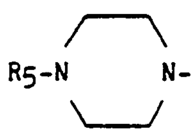

(Y)

(in der $R_5$ eine Pyrimidin-2-yl-gruppe, eine Isochinol-1-yl-gruppe, eine Chinol-2-yl-gruppe, eine Pyrid-2-yl-gruppe, eine Benzylgruppe - die gegebenenfalls durch eine Alkylgruppe mit 1 bis 6 Kohlenstoffatomen, welche ein oder mehrere Halogenatome aufweist, substituiert ist -, eine Thiazol-2-yl-gruppe - die gegebenenfalls durch ein oder mehrere Alkylgruppen mit 1 bis 6 Kohlenstoffatomen oder eine Phenylgruppe substituiert ist -, oder eine Benzothiazol-2-yl-gruppe darstellt), und

- $R_1$ und $R_2$

entweder

gleichartig oder verschieden sind und jeweils eine Phenylgruppe, die durch ein oder mehrere Halogenatome oder durch ein oder mehrere Alkyl- oder Alkoxygruppen mit 1 bis 6 Kohlenstoffatomen substituiert ist,

oder

$R_1$ eine Phenylgruppe und $R_2$ eine Pyrid-2-yl-gruppe (wobei diese beiden Gruppen jeweils durch ein oder mehrere Halogenatome oder ein oder mehrere Alkyl- oder Alkoxygruppen mit 1 bis 6 Kohlenstoffatomen substituiert sein können),

oder

$R_1$ und $R_2$ gemeinsam mit dem Kohlenstoffatom, an das sie gebunden sind, einen Fluorenrest bedeuten,

deren mögliche Stereoisomere und deren Additionssalze mit einer anorganischen oder organischen, pharmazeutisch annehmbaren Säure.

**2.** 3-{2-[4-[Bis(4-fluor-phenyl)-methylen]-piperidino]-ethyl}-8-carbamoyl-4-hydroxy-2-methyl-imidazo[1,5-a]pyrimidin, Verbindung der allgemeinen Formel I nach Anspruch 1, und dessen Additionssalze mit einer anorganischen oder organischen, pharmazeutisch annehmbaren Säure.

**3.** 1-{3-[4-[Bis(4-fluor-phenyl)-methylen]-piperidino]-propyl}-4-(pyrimidin-2-yl)-piperazin, Verbindung der allgemeinen Formel I nach Anspruch 1, und dessen Additionssalze mit einer anorganischen oder organischen, pharmazeutisch annehmbaren Säure.

**4.** 4-(2-Fluor-benzyl)-1-{3-[4-[(4-fluor-phenyl)-phenyl-methylen]-piperidino]-propyl}-piperazin-2,6-dion, Verbindung der allgemeinen Formel I nach Anspruch 1, und dessen Additionssalze mit einer anorganischen oder organischen, pharmazeutisch annehmbaren Säure.

**5.** 1-{3-[4-[(4-Fluor-phenyl)-phenyl-methylen]-piperidino]-propyl]-4-(pyrid-2-yl-methyl)-piperazin-2,6-dion, Verbindung der allgemeinen Formel I nach Anspruch 1, und dessen Additionssalze mit einer anorganischen oder organischen, pharmazeutisch annehmbaren Säure.

**6.** Verfahren zur Herstellung der Verbindungen der allgemeinen Formel I nach Anspruch 1, **dadurch gekennzeichnet**, daß man

**entweder**

entweder

ein Amin der allgemeinen Formel II:

RH (II)

in der R die bezüglich der Formel I in Anspruch 1 angegebenen Bedeutungen besitzt, mit einer Verbindung der allgemeinen Formel III:

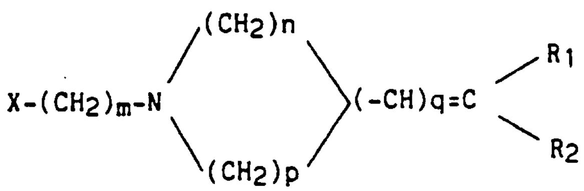

(III)

in der X ein Halogenatom, eine Mesyloxygruppe oder eine Tosyloxygruppe darstellt und m, n, p, q und $R_1$ und $R_2$ die bezüglich der Formel I in Anspruch 1 angegebenen Bedeutungen besitzen, kondensiert zur Bildung der Verbindungen der allgemeinen Formel I nach Anspruch 1,

. oder

ein Amin der Formel IV:

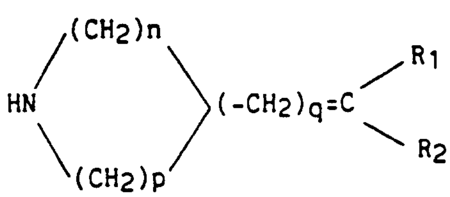

(IV)

in der $R_1$, $R_2$, n, p und q die bezüglich der Formel I in Anspruch 1 angegebenen Bedeutungen besitzen,

mit einer Verbindung der Formel V:

$R - (CH_2)_m - X$ (V)

in der X die bezuglich der Formel III angegebenen Bedeutungen besitzt und R und m die bezuglich der Formel I in Anspruch 1 angegebenen Bedeutungen besitzen, kondensiert zur Bildung der Verbindungen der Formel I,

**oder**

ein 4-Amino-imidazol-Derivat mit einer Verbindung der Formel VI:

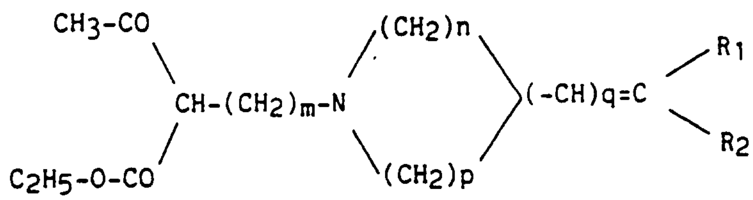

(VI)

in der m, n, p, q, $R_1$ und $R_2$ die bezüglich der Formel I in Anspruch 1 angegebenen Bedeutungen besitzen, cyclisiert,

zur Bildung der Verbindungen der Formel I, in der Reine Imidazo[1,5-a]pyrimidin-gruppe bedeutet und m, n, p, q, $R_1$ und $R_2$ die bezuglich der Formel I in Anspruch 1 angegebenen Bedeutungen besitzen,

welche man anschließend

gewünschtenfalls in ihre möglichen Stereoisomeren auftrennt und/oder mit einer anorganischen oder organischen, pharmazeutisch annehmbaren Säure zur Bildung der entsprechenden Additionssalze in ihre Salze überführt.

**7.** Pharmazeutische Zubereitung enthaltend als Wirkstoff eine Verbindung nach einem der Ansprüche 1 bis 5 in Kombination oder in Mischung mit einem inerten, nichttoxischen, pharmazeutisch annehmbaren Trägermaterial oder Bindemittel.

**8.** Pharmazeutische Zubereitung nach Anspruch 7, enthaltend den Wirkstoff in einer Dosis von 0,5 bis 100 mg.

**9.** Pharmazeutische Zubereitung nach den Ansprüchen 7 und 8, enthaltend als Wirkstoff mindestens eine Verbindung nach den Ansprüchen 1 bis 5 zur Verwendung bei der Behandlung von Krankheiten, welche Antagonisten von Histamin und Serotonin erforderlich machen.

**10.** Amine der Formel $IV_b$:

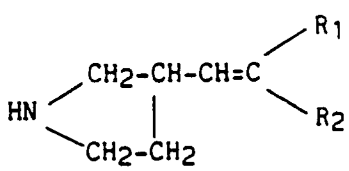

($IV_b$)

in der $R_1$ und $R_2$ die bezuglich der Formel I in Anspruch 1 angegebenen Bedeutungen besitzen, als Zwischenprodukte für die Herstellung der Verbindungen der allgemeinen Formel I nach Anspruch 1.

**11.** Amine der Formel $IV_c$:

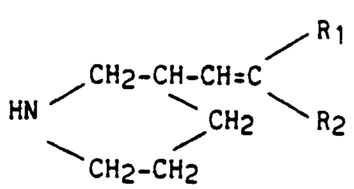

($IV_c$)

in der $R_1$ und $R_2$ die bezüglich der Formel I in Anspruch 1 angegebenen Bedeutungen besitzen, als Zwischenprodukte für die Herstellung der Verbindungen der allgemeinen Formel I nach Anspruch 1.

**12.** Amine der Formel $IV_d$:

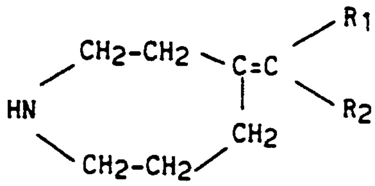

(IVd)

in der $R_1$ und $R_2$ die bezüglich der Formel I in Anspruch 1 angegebenen Bedeutungen besitzen, als Zwischenprodukte für die Herstellung der Verbindungen der allgemeinen Formel I nach Anspruch 1.

**Patentansprüche für folgenden Vertragsstaat : ES**

**1.** Verfahren zur Herstellung von Verbindungen der allgemeinen Formel I:

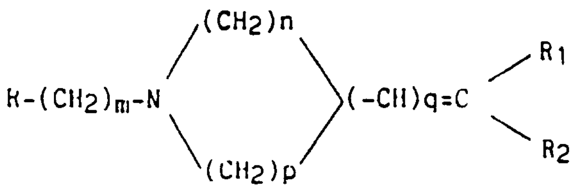

(I)

in der

- m eine ganze Zahl mit einem Wert von 2 bis 4,
- n und p, die gleichartig oder verschieden sein können, ganze Zahlen mit den Werten von 1, 2 oder 3 mit der Maßgabe, daß die Summe von n und p größer oder gleich 3 und kleiner oder gleich 5 ist,
- q 0 oder 1,
- R eine 1,2,3,4-Tetrahydro-chinazol-3-yl-gruppe (die gegebenenfalls am Benzolring durch ein oder mehrere Halogenatome, Alkylgruppen mit 1 bis 6 Kohlenstoffatomen oder Alkoxygruppen mit 1 bis 6 Kohlenstoffatomen substituiert ist), eine 1,3-Dioxo-1,3,4,6,11,11a-hexahydro-2H-pyrazino[1,2-b]isochinol-2-yl-gruppe, eine 1,2-Dihydro-1-oxo-phthalazin-2-yl-gruppe (die gegebenenfalls am Benzolring durch ein oder mehrere Halogenatme, Alkylgruppen mit 1 bis 6 Kohlenstoffatomen oder Alkoxygruppen mit 1 bis 6 Kohlenstoffatomen substituiert ist), eine 2,6-Dioxo-piperazin-1-yl-gruppe der Formel W:

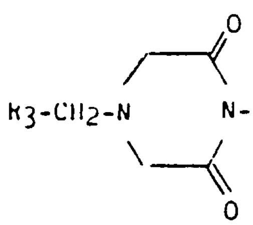

(W)

(in der $R_3$ eine Pyrid-2-yl-gruppe oder eine Phenylgruppe, die gegebenenfalls mit ein oder mehreren Halogenatomen oder Alkylgruppen oder Alkoxygruppen mit 1 bis 6 Kohlenstoffatomen substituiert ist, darstellt),

eine Gruppe der Formel Z:

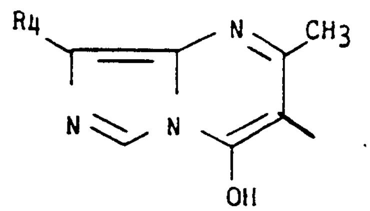

(Z)

(in der $R_4$ eine Carbamoylgruppe, eine Cyanogruppe, eine Hydroxycarbonylgruppe oder eine Alkoxycarbonylgruppe mit 1 bis 6 Kohlenstoffatomen darstellt),
oder eine Gruppe der Formel Y:

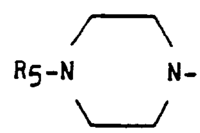

(Y)

(in der $R_5$ eine Pyrimidin-2-yl-gruppe, eine Isochinol-1-yl-gruppe, eine Chinol-2-yl-gruppe, eine Pyrid-2-yl-gruppe, eine Benzylgruppe - die gegebenenfalls durch eine Alkylgruppe mit 1 bis 6 Kohlenstoffatomen, welche ein oder mehrere Halogenatome aufweist, substituiert ist -, eine Thiazol-2-yl-gruppe - die gegebenenfalls durch ein oder mehrere Alkylgruppen mit 1 bis 6 Kohlenstoffatomen oder eine Phenylgruppe substituiert ist -, oder eine Benzothiazol-2-yl-gruppe darstellt), und

- $R_1$ und $R_2$

entweder

gleichartig oder verschieden sind und jeweils eine Phenylgruppe, die durch ein oder mehrere Halogenatome oder durch ein oder mehrere Alkyl- oder Alkoxygruppen mit 1 bis 6 Kohlenstoffatomen substituiert ist,

oder

$R_1$ eine Phenylgruppe und $R_2$ eine Pyrid-2-yl-gruppe (wobei diese beiden Gruppenjeweils durch ein oder mehrere Halogenatome oder ein oder mehrere Alkyl- oder Alkoxygruppen mit 1 bis 6 Kohlenstoffatomen substituiert sein können),

oder

$R_1$ und $R_2$ gemeinsam mit dem Kohlenstoffatom, an das sie gebunden sind, einen Fluorenrest bedeuten,

und von deren möglichen Stereoisomeren und deren Additionssalzen mit einer anorganischen oder organischen, pharmazeutisch annehmbaren Säure,

**dadurch gekennzeichnet**, daß man

**entweder**

entweder

ein Amin der allgemeinen Formel II:

RH (II)

in der R die bezüglich der Formel I angegebenen Bedeutungen besitzt,
mit einer Verbindung der allgemeinen Formel III:

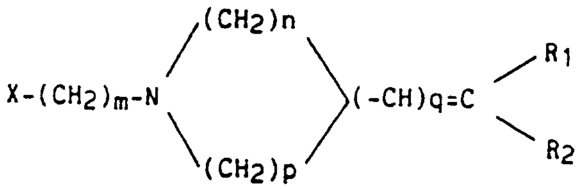

(III)

in der X ein Halogenatom, eine Mesyloxygruppe oder eine Tosyloxygruppe darstellt und m, n, p, q und $R_1$ und $R_2$ die bezüglich der Formel I angegebenen Bedeutungen besitzen, kondensiert zur Bildung der Verbindungen der allgemeinen Formel I,

. oder
ein Amin der Formel IV:

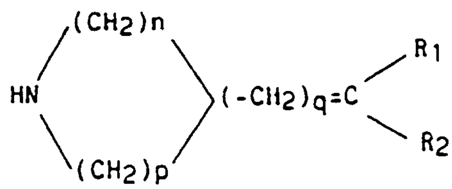

(IV)

in der $R_1$, $R_2$, n, p und q die bezüglich der Formel I angegebenen Bedeutungen besitzen, mit einer Verbindung der Formel V:

$R - (CH_2)_m - X$ (V)

in der X die bezüglich der Formel III angegebenen Bedeutungen besitzt und R und m die bezüglich der Formel I angegebenen Bedeutungen besitzen, kondensiert zur Bildung der Verbindungen der Formel I,

**oder**

ein 4-Amino-imidazol-Derivat mit einer Verbindung der Formel VI:

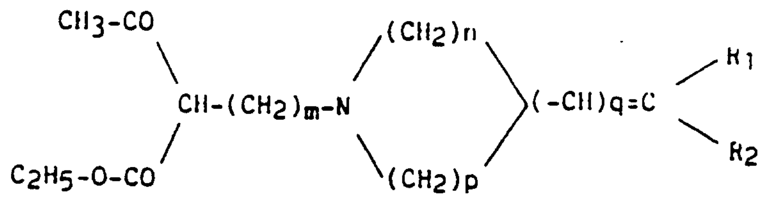

(VI)

in der m, n, p, q, $R_1$ und $R_2$ die bezüglich der Formel I angegebenen Bedeutungen besitzen, cyclisiert,

zur Bildung der Verbindungen der Formel I, in der Reine Imidazo[1,5-a]pyrimidin-gruppe bedeutet und m, n, p, q, $R_1$ und $R_2$ die bezüglich der Formel I angegebenen Bedeutungen besitzen,

**welche man anschließend**

gewünschtenfalls in ihre möglichen Stereoisomeren auftrennt und/oder mit einer anorganischen oder organischen, pharmazeutisch annehmbaren Saure zur Bildung der entsprechenden Additionssalze in ihre Salze überführt.

**2.** 3-{2-[4-[Bis(4-fluor-phenyl)-methylen]-piperidino]-ethyl}-8-carbamoyl-4-hydroxy-2-methyl-imidazo[1,5-a]pyrimidin, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**3.** 1-{3-[4-[Bis(4-fluor-phenyl)-methylen]-piperidino]-propyl}-4-(pyrimidin-2-yl)-piperazin, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**4.** 4-(2-Fluor-benzyl)-1-{3-[4-[(4-fluor-phenyl)-phenyl-methylen]-piperidino]-propyl}-piperazin-2,6-dion, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**5.** 1-{3-[4-[(4-Fluor-phenyl)-phenyl-methylen]-piperidino]-propyl]-4-(pyrid-2-yl-methyl)-piperazin-2,6-dion, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**6.** Amine der Formel $IV_b$:

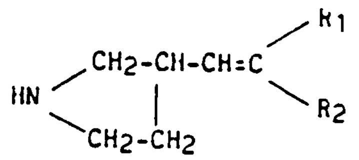

(IVb)

in der $R_1$ und $R_2$ die bezuglich der Formel I in Anspruch 1 angegebenen Bedeutungen besitzen, als Zwischenprodukte für die Herstellung der Verbindungen der allgemeinen Formel I nach Anspruch 1.

**7.** Amine der Formel $IV_c$:

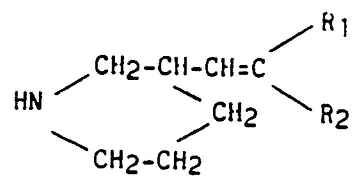

(IVc)

in der $R_1$ und $R_2$ die bezuglich der Formel I in Anspruch 1 angegebenen Bedeutungen besitzen, als Zwischenprodukte für die Herstellung der Verbindungen der allgemeinen Formel I nach Anspruch 1.

**8.** Amine der Formel $IV_d$:

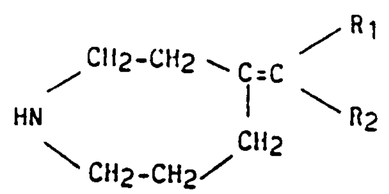

(IVd)

in der $R_1$ und $R_2$ die bezuglich der Formel I in Anspruch 1 angegebenen Bedeutungen besitzt, als Zwischenprodukte für die Herstellung der Verbindungen der allgemeinen Formel I nach Anspruch 1.

**Patentansprüche für folgenden Vertragsstaat : GR**

**1.** Verfahren zur Herstellung von Verbindungen der allgemeinen Formel I:

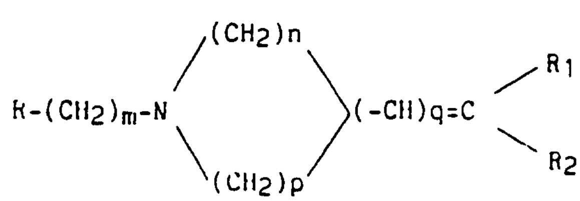

(I)

in der

- m eine ganze Zahl mit einem Wert von 2 bis 4,
- n und p, die gleichartig oder verschieden sein können, ganze Zahlen mit den Werten von 1, 2 oder 3 mit der Maßgabe, daß die Summe von n und p größer oder gleich 3 und kleiner oder gleich 5 ist,
- q 0 oder 1,
- Reine 1,2,3,4-Tetrahydro-chinazol-3-yl-gruppe (die gegebenenfalls am Benzolring durch ein oder mehrere Halogenatome, Alkylgruppen mit 1 bis 6 Kohlenstoffatomen oder Alkoxygruppen mit 1 bis 6 Kohlenstoffatomen substituiert ist), eine 1,3-Dioxo-1,3,4,6,11,11a-hexahydro-2H-pyrazino[1,2-b]isochinol-2-yl-gruppe, eine 1,2-Dihydro-1-oxo-phthalazin-2-yl-gruppe (die gegebenenfalls am Benzolring durch ein oder mehrere Halogenatme, Alkylgruppen mit 1 bis 6 Kohlenstoffatomen oder Alkoxygruppen mit 1 bis 6 Kohlenstoffatomen substituiert ist), eine 2,6-Dioxo-piperazin-1-yl-gruppe der Formel W:

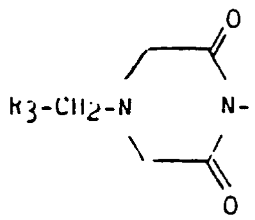

(W)

(in der $R_3$ eine Pyrid-2-yl-gruppe oder eine Phenylgruppe, die gegebenenfalls mit ein oder mehreren Halogenatomen oder Alkylgruppen oder Alkoxygruppen mit 1 bis 6 Kohlenstoffatomen substituiert ist, darstellt),

eine Gruppe der Formel Z:

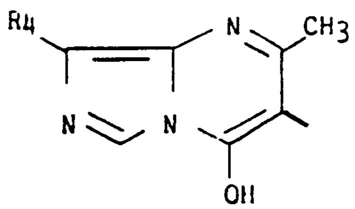

(Z)

(in der $R_4$ eine Carbamoylgruppe, eine Cyanogruppe, eine Hydroxycarbonylgruppe oder eine Alkoxycarbonylgruppe mit 1 bis 6 Kohlenstoffatomen darstellt),

oder eine Gruppe der Formel Y:

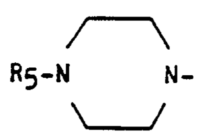

(Y)

(in der $R_5$ eine Pyrimidin-2-yl-gruppe, eine Isochinol-1-yl-gruppe, eine Chinol-2-yl-gruppe, eine Pyrid-2-yl-gruppe, eine Benzylgruppe - die gegebenenfalls durch eine Alkylgruppe mit 1 bis 6 Kohlenstoffatomen, welche ein oder mehrere Halogenatome aufweist, substituiert ist -, eine Thiazol-2-yl-gruppe - die gegebenenfalls durch ein oder mehrere Alkylgruppen mit 1 bis 6 Kohlenstoffatomen oder eine Phenylgruppe substituiert ist -, oder eine Benzothiazol-2-yl-gruppe darstellt), und

- $R_1$ und $R_2$

entweder

gleichartig oder verschieden sind und jeweils eine Phenylgruppe, die durch ein oder mehrere Halogenatome oder durch ein oder mehrere Alkyl- oder Alkoxygruppen mit 1 bis 6 Kohlenstoffatomen substituiert ist,

oder

$R_1$ eine Phenylgruppe und $R_2$ eine Pyrid-2-yl-gruppe (wobei diese beiden Gruppen jeweils durch ein oder mehrere Halogenatome oder ein oder mehrere Alkyl- oder Alkoxygruppen mit 1 bis 6 Kohlenstoffatomen substituiert sein können),

oder

$R_1$ und $R_2$ gemeinsam mit dem Kohlenstoffatom, an das sie gebunden sind, einen Fluorenrest bedeuten,

und von deren möglichen Stereoisomeren und deren Additionssalzen mit einer anorganischen oder organischen, pharmazeutisch annehmbaren Säure,

**dadurch gekennzeichnet**, daß man

**entweder**

entweder
ein Amin der allgemeinen Formel II:

RH (II)

in der R die bezüglich der Formel I angegebenen Bedeutungen besitzt, mit einer Verbindung der allgemeinen Formel III:

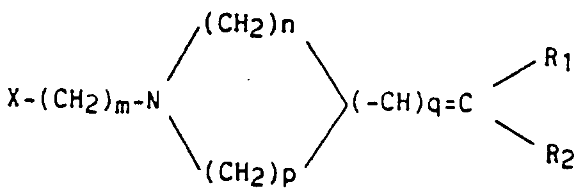

(III)

in der X ein Halogenatom, eine Mesyloxygruppe oder eine Tosyloxygruppe darstellt und m, n, p, q und $R_1$ und $R_2$ die bezüglich der Formel I angegebenen Bedeutungen besitzen, kondensiert zur Bildung der Verbindungen der allgemeinen Formel I,
. oder
ein Amin der Formel IV:

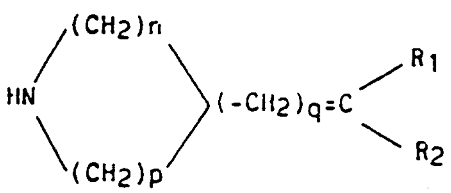

(IV)

in der $R_1$, $R_2$, n, p und q die bezüglich der Formel I angegebenen Bedeutungen besitzen, mit einer Verbindung der Formel V:

R - $(CH_2)_m$ - X (V)

in der X die bezüglich der Formel III angegebenen Bedeutungen besitzt und R und m die bezüglich der Formel I angegebenen Bedeutungen besitzen, kondensiert zur Bildung der Verbindungen der Formel I,
**oder**
ein 4-Amino-imidazol-Derivat mit einer Verbindung der Formel VI:

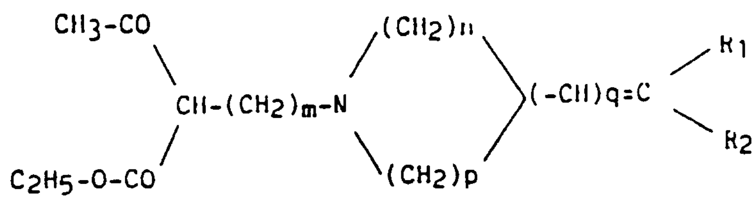

(VI)

in der m, n, p, q, $R_1$ und $R_2$ die bezüglich der Formel I angegebenen Bedeutungen besitzen, cyclisiert,
zur Bildung der Verbindungen der Formel I, in der Reine Imidazo[1,5-a]pyrimidin-gruppe bedeutet und m, n, p, q, $R_1$ und $R_2$ die bezüglich der Formel I angegebenen Bedeutungen besitzen,
**welche man anschließend**
gewünschtenfalls in ihre möglichen Stereoisomeren auftrennt und/oder mit einer anorganischen oder organischen, pharmazeutisch annehmbaren Säure zur Bildung der entsprechenden Additionssalze in ihre Salze überführt.

**2.** 3-{2-[4-[Bis(4-fluor-phenyl)-methylen]-piperidino]-ethyl}-8-carbamoyl-4-hydroxy-2-methyl-imidazo[1,5-a]pyrimidin, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**3.** 1-{3-[4-[Bis(4-fluor-phenyl)-methylen]-piperidino]-propyl}-4-(pyrimidin-2-yl)-piperazin, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**4.** 4-(2-Fluor-benzyl)-1-{3-[4-[(4-fluor-phenyl)-phenyl-methylen]-piperidino]-propyl}-piperazin-2,6-dion, Verbindung der allgemeinen Formel I nach Anspruch 1, und dessen Additionssalze mit einer anorganischen oder organischen, pharmazeutisch annehmbaren Säure, hergestellt nach dem Verfahren nach Anspruch 1 oder einem chemisch äquivalenten Verfahren.

**5.** 1-{3-[4-[(4-Fluor-phenyl)-phenyl-methylen]-piperidino]-propyl]-4-(pyrid-2-yl-methyl)-piperazin-2,6-dion, Verbindung der allgemeinen Formel I nach Anspruch 1, und dessen Additionssalze mit einer anorganischen oder organischen, pharmazeutisch annehmbaren Säure, hergestellt nach dem Verfahren nach An-spruch 1 oder einem chemisch äquivalenten Verfahren.

**6.** Amine der Formel $IV_b$:

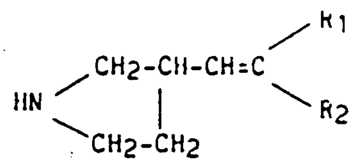

($IV_b$)

in der $R_1$ und $R_2$ die bezüglich der Formel I in Anspruch 1 angegebenen Bedeutungen besitzen, als Zwischenprodukte für die Herstellung der Verbindungen der allgemeinen Formel I nach Anspruch 1.

**7.** Amine der Formel $IV_c$:

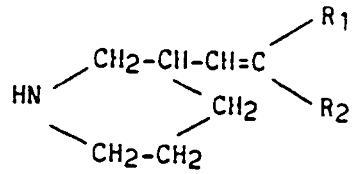

($IV_c$)

in der $R_1$ und $R_2$ die bezüglich der Formel I in Anspruch 1 angegebenen Bedeutungen besitzen, als Zwischenprodukte für die Herstellung der Verbindungen der allgemeinen Formel I nach Anspruch 1.

**8.** Amine der Formel $IV_d$:

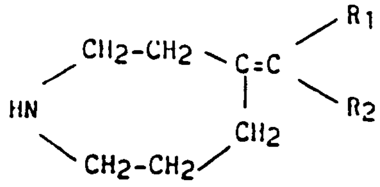

($IV_d$)

in der $R_1$ und $R_2$ die bezüglich der Formel I in Anspruch 1 angegebenen Bedeutungen besitzt, als Zwischenprodukte für die Herstellung der Verbindungen der allgemeinen Formel I nach Anspruch 1.